# EUROPEAN PATENT SPECIFICATION

(11) **EP 3 631 446 B1**
(45) Date of publication and mention of the grant of the patent: **17.04.2024**
(21) Application number: 18727307.3
(22) Date of filing: 29.05.2018
(51) Int. Cl.: G01N 33/52, G01N 21/64, G01N 21/3577, G01N 21/552, G01N 21/39

(54) **BIOSENSOR FOR CONFORMATION AND SECONDARY STRUCTURE ANALYSIS**
BIOSENSOR ZUR KONFORMATION UND SEKUNDÄREN STRUKTURANALYSE
BIOCAPTEUR POUR LA CONFORMATION ET L'ANALYSE DE STRUCTURE SECONDAIRE

(30) Priority: 29.05.2017 EP 17173322
(43) Date of publication of application: 08.04.2020
(73) Proprietor: betaSENSE GmbH, 44799 Bochum (DE)
(72) Inventor: GERWERT, Klaus, 44799 Bochum (DE)
(74) Representative: Maiwald GmbH
(86) International application number: PCT/EP2018/064103
(87) International publication number: WO 2018/219969

(56) References cited:
- WO-A1-2015/121339
- J. LIN ET AL: "A Modified Infrared Spectrometer with High Time Resolution and Its Application for Investigating Fast Conformational Changes of the GTPase Ras", APPLIED SPECTROSCOPY (SOCIETY FOR APPLIED SPECTOSCOPY), vol. 68, no. 5, 1 May 2014 (2014-05-01), pages 531-535, XP055399757, Frederick, MD 21703, USA ISSN: 0003-7028, DOI: 10.1366/13-07320

## Description

The invention provides an infrared detection system for conformation and secondary structure analysis, notably for the direct non-invasive qualitative secondary structure analysis of a single selected protein within a complex mixture, as e.g. a body fluid, by vibrational spectroscopic methods utilizing a quantum- cascade laser. For the analysis it is not required that the selected substance be isolated, concentrated, or pretreated by a special preparative procedure. A difference-spectrum between the unbound and antibody-bound protein of interest is performed by which the much larger background absorbance is cancelled. The presented quantum- cascade laser set-up provides sufficient S/N and stability to subtract the several orders of magnitude larger background absorbance.

### Background of the Invention

Quantitative methods for the detection of biomarker candidates in bodily fluids are enzyme-linked immune-sorbent assays (ELISA), surface plasmon resonance spectroscopy (SPR), surface fluorescence intensity distribution analysis (sFIDA) or mass spectroscopy techniques. These techniques do not provide direct information about the secondary structure of the analytes. Antibody based methods like ELISA or SPR may be complemented with conformation sensitive antibodies, so that structure information of particularly one conformation can be derived indirectly (I. Morgado et al., Proc. Natl. Acad. Sci., 109(31): 12503-12508 (2012); Venkataramani et al., JAD 29(2):361-371 (2012)). Using these conformation sensitive antibodies, our studies demonstrated that only the specific secondary structure was detected. Thus, a sample discrimination based on the specific structural composition of an analyzed compound is not possible; all detectable conformations are present in natural samples, but the composition varies in e.g. a disease. The later described discrimination requires a structure independent antibody, because the recorded signal has to reflect concentration differences of the observed structures. sFIDA detects candidate biomarker dimers or oligomers by using identical immobilization and detection antibodies. However, sFIDA does not detect structure information (S. Funke et al., Rejuvenation Res., 13(2-3):206-209 (2010)). The secondary structure analysis of proteins by Fourier-transform infrared (FTIR-) spectroscopy and the analysis of recombinant or purified proteins after immobilization on particular attenuated total reflection (ATR-) sensor surfaces has frequently been described (J. Ollesch et al., Appl. Spectrosc., 61(10):1025-1031 (2007); K. Elfrink, J. Ollesch et al., Proc Natl Acad Sci, 105(31):10815-10819 (2008); Frost et al., J. Biol. Chem., 284(6):3546-3551 (2009); S. Funke et al., J. Biol. Chem., 280(10):8912-7 (2005)). FTIR-spectroscopic secondary structure analysis of nucleic acids like RNA have already been published (E. Brauns and R. B. Dyer, Biophys. J., 89(5):3523-3530 (2005)). In the present state of scientific and technical knowledge no secondary structure analysis of components from complex fluids like serum, blood plasma or cerebrospinal fluid without prior isolation have been reported to date. The selective detection of specific components out of a complex body fluid by applying an ATR-flow-through sensor constitutes an innovative new development. So far this technique was only applied to isolated proteins. Internal reflection elements (IRE) of ATR-sensors typically consist of infrared permeable materials with a high refraction index. These include diamond, germanium, silicon or zinc selenide. Proteins are immobilized on these surfaces via tethered lipids (K. Elfrink, L. Nagel-Steger and D. Riesner, Biol. Chem., 388(1):79-89 (2007); K. Elfrink, J. Ollesch et al., PNAS 2008; J. Güldenhaupt et al., The FEBS Journal, 275(23):5910-5918 (2008); C. Kötting et al., Chemical Physics, 396:72-83 (2012); P. Pinkerneil et al., Chemphyschem, 13(11):2649-2653 (2012)), thiolchemistry on vapor-deposited or chemical secluded gold surfaces (Ataka et al., J. Am. Chem. Soci., 126(49): 16199-16206 (2004); A. Badura et al., Photochem. and Photobiol., 82(5): 1385-1390 (2006)) or silanes (B. M. Smith et al., Langmuir, 20(4):1184-1188 (2004); S. Devouge et al., Bioorg. & Med. Chem. Lett., 15(13):13252-13256 (2005); P. W. Loscutoff and S. F. Bent, Ann. Rev. Physical Chemistry, 57(1):467-495 (2006); J. Matijasević et al., Langmuir, 24(6):2588-2596 (2008); S. Devouge et al., Journal of Colloid and Interface Science, 332(2):408-415 (2009); J. Schartner et al., J. Am. Chem. Soci., 135(10):4079-4087 (2013)). In this process, the immobilization of antibodies or other proteins on other semiconductors than germanium has been described (P. Hofer and Fringeli, Biophysics of Structure and Mechanism, 6(1):67-80 (1979); S. Löfas and B. Johnsson, J. Chem. Soc., Chem. Comm. (21):1526 (1990); B. Byrne et al., Sensors (Basel, Switzerland), 9(6):4407-4445 (2009); M. Punzet et al., Nanoscale, 4(7):2431 (2012)). Invention relevant reagents have been synthesized by the inventers. The basic silanes were published (J. Schartner et al., J. Am. Chem. Soc., 135(10):4079-4087 (2013)), but the main application, the immobilization of antibodies through free lysine residues and short chain triethoxysilanes, has not been described so far. The antibody immobilization through proteinogenic lysines on other succinimidylester has been reported (S. Löfas and B. Johnsson, J. Chem. Soci., Chem. Comm. (21):1526 (1990); EP-B-1214594 and WO2000070345). However, the analysis was not performed by IR spectroscopy, therefore protein secondary structure analysis was not performed. The use of functionalized short-chain trialkoxysilanes (such as N-(4,4,4-triethoxysilanebutyl)succinamic acid 2,5-dioxopyrrolidin-1-yl ester) for covalent protein immobilization has not been reported.

In a further approach, the ATR-IRE were silanized and coupled with biotin resulting in an avidin/streptavidin sensor without any secondary structure analysis (M. Voue et al., Langmuir, 23(2):949-955 (2007)). Here the sensor surface was modified in a more complex workflow and through aggressive chemicals, which can influence and change the secondary structure of the analyte. It was shown that the presented preparation is not appropriate to generate the proposed sensor for the analysis of a selected protein in a complex bodyfluid under physiological conditions (Kleiren et al., Spectroscopy- An International Journal, 24 (1-2, SI): 61-66. (2010)). Apart from Voue (M. Voue et al., Langmuir, 23(2):949-955 (2007); S. Devouge et al., Journal of Colloid and Interface Science, 332(2):408-415 (2009)), 02/056018 and EP-A-1806574 disclose an optical element suitable for the analysis of ligand-receptor interactions.

WO 02/056018 refers explicitly to a device for the investigation of ligand interactions with a receptor, consisting of an attenuated total internal reflection element, transparent in the infrared and of which at least one surface is chemically activated by oxidation, hydroxylation or reduction and covalently grafted with a long chain silane derivative capable of immobilizing the receptor. The attenuated total internal reflection element is made from a material selected from germanium, silicon, ZnSe, ZnS, and AM-TIR. The device is suitable for studying ligand-receptor interactions. Further WO 02/001202 mentions the combination of ATR-IR-spectroscopy with polarized radiation and refractometric measurements.

EP-A-1806574 discloses a device suitable for the investigation of ligand-receptor interactions, in particular for the investigation of an analyte-target interaction such as biological and chemical molecules and organic components and their interaction with surfaces, consisting of an attenuated total internal reflection element, transparent in the infrared and of which at least one surface is reduced and covalently grafted with an alkene able to immobilize the receptor, wherein said alkene is optionally substituted by one or more substituent selected from alkyl, haloalkyl, halo, alkenyl, cyano, epoxy, thio, amino, hydroxyl, isocyano, isothiocyano, carboxy, polyalkoxy, alkylarylsulphoxy-polyalkoxy, or heteroaryloxycarbonylakyl-polyalkoxy. The attenuated total internal reflection element is made from germanium, notably a crystal having a trapezoidal, hemi - cylindrical, fiber or rod shaped geometry, or polyhedral form. The device is suitable for studying ligand-receptor interactions, in particular biological molecules or organic components or their interactions or complexations or reactions with biological molecules or organic components or watersoluble molecules at or in the grafted organic molecule.

WO 02/001202 and US2012/0309943 discloses the principal generation of an antibody-support by, for example, silanes.

WO 07/131997 refers to an ATR-IR-measurement setup, in which the sample is sustained in a specified distance to the ATR-surface without direct contact. A spectroscopically inert medium is intended as spacer.

Conventional spectroscopy requires a multistage preparation of complex samples, to isolate the single analyte in a high concentration for analysis. Secondary structures may change during preparation.

SPR and ELISA methods quantify specific components with high sensitivity in complex media, but cannot gather secondary structure information. These are highly sensitive, but purely quantitative methods. Conformationally sensitive (implying conformational specificity) antibodies are generally insensitive for transition states of the analyte structure (S. A. Funke, International Journal of Alzheimer's Disease, 2011:1-8 (2011); K. A. Bruggink et al., Analytical Biochemistry, 433(2):112-120 (2013)), which are nevertheless relevant for a disease (I. Benilova et al., Nature Neuroscience, 15(3):349-357 (2012)).

IR compatible materials reported for antibody binding comprise silicon, diamond and germanium. Silicon absorbs IR radiation in the analyzed spectral fingerprint range. Diamond is an expensive material which prevents the realization of larger detector areas for an increased sensitivity. The refractive indices of silicon and diamond are lower than of germanium, which reflects in a decreased signal/noise ratio as compared to the latter.

By the selection of identical antibodies for capture and detection, sFIDA is sensitive for di- or oligomeric aggregates (L. Wang-Dietrich et al., JAD, 34(4):985-994 (2013)). The secondary structure is not directly analyzed.

The secondary structure analysis of proteins is a standard application of an array of techniques (UV/Vis circular dichroism spectroscopy, IR spectroscopy, NMR spectroscopy). Altogether, highly pure and concentrated proteins are required for analysis.

Protein immobilization via silanes is disclosed in EP-A-1806574 and WO 02/056018 for the analysis of receptor-ligand interactions exclusively. Thus, reactions of and with the tethered protein were considered. The secondary structure analysis of further ligands of the tethered proteins was not considered.

A reliable diagnosis of the most relevant known protein misfolding disease, Alzheimer's, currently requires an advanced state of the disease. Current biomarker analysis is based on quantitative ELISA. The structural transition of e.g. the amyloid-beta (Aβ) peptide during disease progression is thought to be initiated long before clinical symptoms of the patient. This considered, the structural analysis of the biomarker candidate not only offers potential for supplementing established diagnostics, but - even more important - may enable an earlier timepoint for diagnosis. Thus, a therapy may start earlier, securing longer life quality.

In applicants WO 2015/121339 the direct secondary structure analysis of selective components from a complex body fluid without prior isolation or concentration. It is based on a sensor element having antibodies directly immobilized thereon via short silane or thiol linkers, notably a germanium surface where the antibodies are bound covalently via a peptide bond to immobilized triethoxysilane or thiol linkers. The immunological linkage renders the germanium surface highly specific for selective substances, similar to ELISA methods. The captured substances are analyzed by infrared spectroscopy for the particular secondary structure. The potentially prognostic misfolding can be quantified. With the method the biomarker secondary structure within a complex body fluid can be specified. The sensor design enables a parallel control with an alternative spectroscopic technique, e.g. fluorescence spectroscopy. The immunologically determined high specificity for a substance enables the direct secondary structure analysis of selected biomarkers from complex fluids as e.g. cerebrospinal fluid (csf) or blood without pretreatment.

In WO 2015/121339 any IR-light source can be used for the sensing IR beam guided through the internal reflection element of the ATR-cell with the antibody capture surface.

On the other hand, J. Lin et al., Applied Spectroscopy 68(5):531-535 (2014) reports on conformational changes of the GTPase Ras with a modified IR-spectrometer with high time resolution and emphasizes that the time resolution might be further improved by utilizing a quantum-cascade laser.

### Short Description of the invention

The subject matter of the invention is as defined in the appended claims.

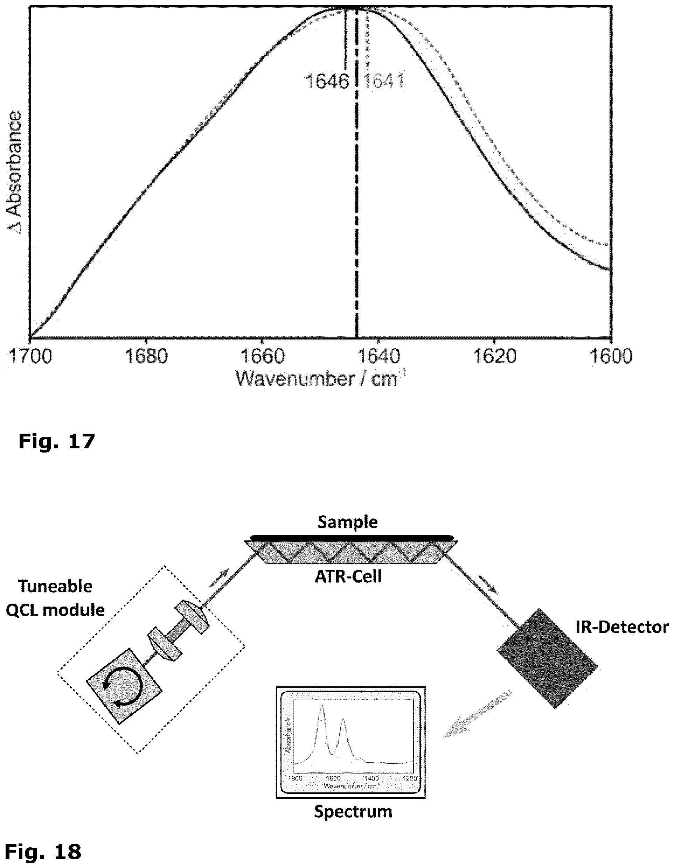

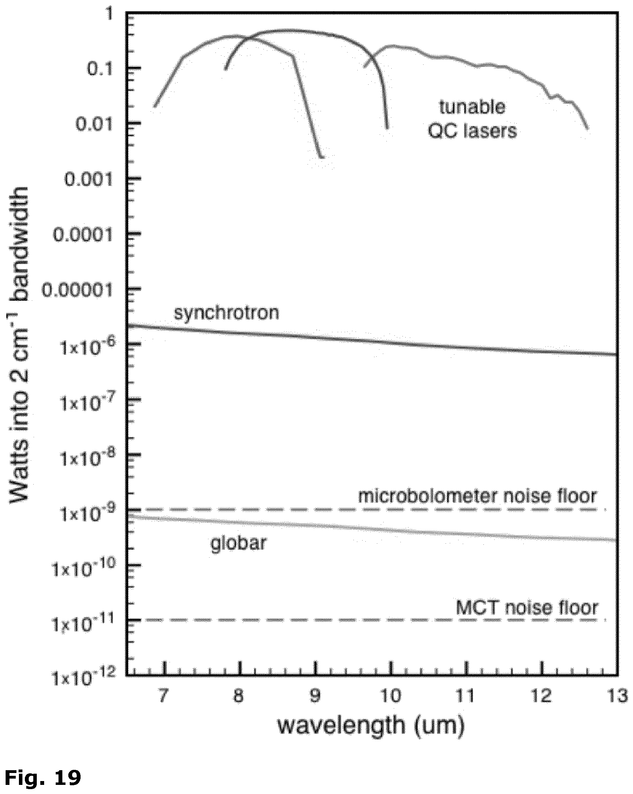

It was now found that the use of a tunable quantum-cascade laser as the IR source together with the above mentioned biosensor of as illustrated in Figure 18 provides for enhanced sensitivity of the detection system. This because quantum cascade lasers deliver much higher light intensities as compared to other IR-sources (fig 19). Therefore, the measuring time is expected to be significantly reduced at an equivalent SNR. Since QCLs are much smaller than conventional FTIR spectrometers a very compact instrument can be realized. Due to the high power density of the light source even Peltier-cooled MCT or microbolometers can be used as detectors. Thereby the QCL based set-up is much better suited for a clinical application than conventional FTIR-set-ups.

The invention thus provides:
(1) An infrared detection system for the direct analysis of the quantity and secondary structure of a candidate biomarker protein undergoing conformational transitions associated with disease progression, said detection system comprising:
   (a) a tunable quantum-cascade laser as the IR source, and
   (b) an IR cell with an infrared sensor element that comprises a germanium internal reflection element being of trapezoid or parallelogram shape, which allows for more than one passages of the infrared light through the reflection element, and being transparent in the infrared with sufficient signal to noise ratio to detect the amide I band, and at least one receptor for the biomarker protein being an antibody capable of specific and conformationally independent binding to the candidate biomarker protein and being directly grafted to at least one surface of said internal germanium reflection element by silanization with short silane linkers or by thiolation with short thiol linkers, reacting freely accessible amine groups of said at least one receptor with amine-reactive groups on the short silane/thiol linkers, and blocking remaining amine-reactive groups on the short silane/thiol linkers with a blocking substance not cross-reacting with the candidate biomarker protein.
(2) The Use of the infrared detection system of (1) above for determining the secondary structure, and optionally the quantity, of a candidate biomarker protein undergoing conformational transitions associated with disease progression in a complex fluid including bodily fluids.
(3) A method for determining the secondary structure, and optionally the quantity, of a candidate biomarker protein undergoing conformational transitions associated with disease progression in a complex fluid, comprising the steps
   (a) conducting, in the IR cell comprising the infrared detection system of (1), a flux of potential candidate biomarker proteins for the receptor on the surface of said infrared sensor;
   (b) submitting an IR beam through said cell with the tunable quantum-cascade laser and obtaining an infrared spectrum therefrom; and
   (c) analyzing the obtained infrared spectrum to determine the secondary structure, and optionally the quantity, of the candidate biomarker protein.

### Short Description of the Figures

Fig. 1: Schematic view of the sensoric device in the sample chamber of an IR spectrometer (A), detailed view on the sample chamber (B), and schematics of the flow through cuvette (C).
Fig. 2: Optimized flow through cuvette in detail. The device is prepared for a parallel analysis with alternative optical technique via a quartz window in the cover. Gasket elements, Inlet, and outlet ports were optimized regarding stability and flow.
Fig. 3: Short chain triethoxysilane (N-(4,4,4-Triethoxysilanebutyl)succinamic Acid 2,5-Dioxopyrrolidin-1-yl Ester) was covalently attached to germanium (A). The succinimidyl ester reacts with free amines of e.g. proteinogenic lysines, which leads to a stable attachment of the desired protein, *e.g*. an antibody, of which the attached lysine side chain is shown (B). As alternative linker, 12-mercaptododecanoic acid NHS ester was also covalently attached to germanium (C). The NHS ester reacts with free amines of e.g. proteinogenic lysines, also forming a covalent bond (D).
Fig. 4: Fluorescence microscopical analysis of the experiment. On reactive silane (A), no fluorescence was detected (B), but 8G7-FITC antibodies were bound (C). Binding of the detection antibody 1E8 (D) does not increase fluorescence (E). The casein blocked surface (F) does neither show fluorescence (G) nor bind detection antibody 8G7-FITC (H). Only specifically immobilized Aβ-peptide (here: Aß₁₋₄₂, I) captures antibody 8G7-FITC (J).

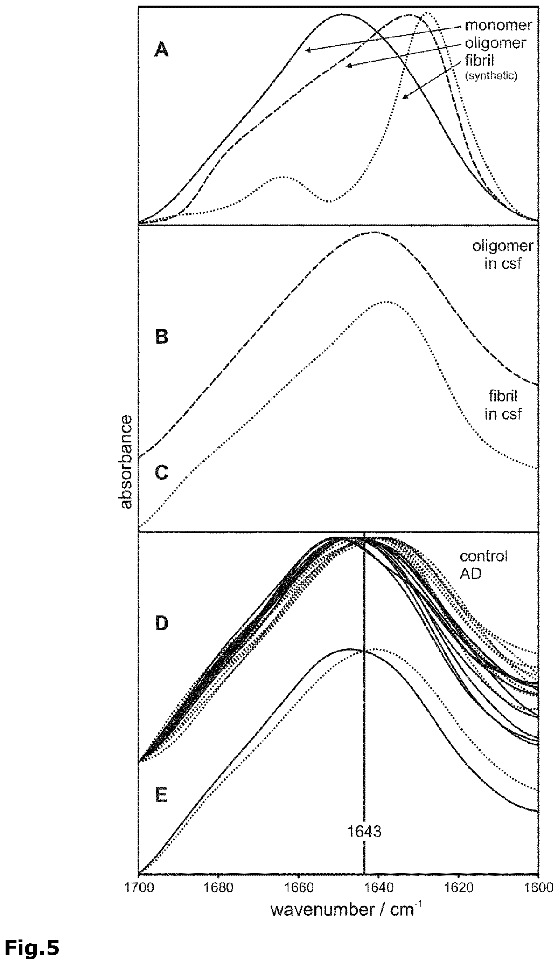

Fig. 5: The amide I marker bands of the infrared absorbance of Aβ peptides, that are used for discrimination of non-neurodegenerative control patients from Alzheimer disease patients with our invention. Synthetic, isolated Aβ peptides in "healthy", monomeric (solid line), "disease" oligomeric (dashed) and "disease" fibrillar conformation (dotted) (A) were captured by antibody 1E8 to the ATR surface. The secondary structure composition unambiguously differs. The amide I band of the oligomeric Aβ fraction from human csf captured with antibody KW1 (B, dashed) differs from the native fibrillar fraction as captured with antibody B10 (C, dotted). Both reflect the natural conformational variety as compared to synthetic samples. The Aβ fractions from unprocessed csf of 14 control (solid) and 9 Alzheimer disease patients (dotted), captured with conformationally independent antibody A8978 (D), differ less than synthetic peptides in defined conformations. Nevertheless, the separation is unique: all control amide I maxima are above 1643 cm⁻¹, all Alzheimer patients below (separating line). Even more pronounced, the arithmetic average of the class spectra indicates the different band positions (E).
Fig. 6: The amide I band position enables a unique discrimination of control from Alzheimer disease patients (A). A classifying LDA with quadratic separation function calculated with extinction values at 1647 and 1640 cm⁻¹ confirmed these findings (B).
Fig. 7: The amide I band maxima of the Aß peptide fractions of EDTA stabilized blood plasma obtained from a control and an Alzheimer disease patient discriminate identically to Aß of csf (Fig. 5E). Antibody A8978 was linked via 12-mercaptododecanoic acid NHS ester to the Ge-IRE.
Fig. 8: Signal to noise (S/N) ratio of amide I (black squares) and amide II bands (grey circles) of synthetic Aβ peptides.
Fig. 9: The average amide I bands of 37 AD and 63 non-neurodegenerative control patients as detected from CSF (A) indicate a clear frequency downshift in AD patient samples. Using a frequency threshold as discriminator, the ROC curve indicated an AUC of 0.93 (B).
Fig. 10: The average amide I bands of 35 AD and 61 non-neurodegenerative control patients as detected from blood plasma (A) indicate a similar frequency downshift in AD patient samples as detected in CSF. Using a frequency threshold as discriminator, the ROC curve indicated an AUC of 0.85 (B).
Fig. 11: Distribution of the amide I band maximum positions recorded of CSF (A) and blood plasma samples (B). Solid diamonds depict control patient samples, empty diamonds AD cases. Gaussian normal distributions well approximated the displayed histogram data. 25/50/75 % quantiles are displayed in box-plots. These further indicate the average band position (square), ± standard deviation (SD, whiskers), and observed minimum/maximum values (x). A dashed line indicates the discriminative threshold position of 1643 cm⁻¹.
Fig. 12: The amide I band as detected from CSF of two patients with the sensor prepared with thiol linkers instead of silane linkers of the mAB A8978 exhibits identical band features for disease class separation (dash-dotted line).
Fig. 13: (A) Unblocked sensors readily bound alpha-synuclein or albumin from pure samples (separate sensor elements) and remained receptive for Aβ peptides from a pure solution in serial application of these proteins (dotted: combined signal of albumin and alpha synuclein). (B) A blocked sensor exhibited no detectable binding of alpha-synuclein and albumin. Only Aß peptides were detected after serial application.
Fig. 14: Amide I and II bands obtained with a blocked (dashed, grey) and an unblocked sensor element (solid, black) after incubation with CSF of a confirmed AD patient.
Fig. 15: Amide I and II bands obtained with the sensor after incubation with conditioned cell culture medium and consecutive rinsing. Blocking of the surface with casein resulted in the expected band pattern and intensities of mostly alpha helical Aβ peptides (A). Using albumin instead of casein, an increased signal intensity and an irregular band pattern indicate overlaying spectral contributions of undesired substances (B).
Fig. 16: Amide I bands obtained with different antibodies on a blocked sensor from the Aß fraction in CSF of control (solid, black) and AD patients (dashed, grey). 1E8 recognized an N-terminal epitope (A). KW1 captured oligomeric Aβ peptides (B). B10 selected fibrils (C).
Fig. 17: The blood serum-borne alpha-synuclein fraction as captured with antibody 4B12 exhibited a similar conformational transition in blood samples of control (black, solid) and PD patients (grey, dashed line) dividable by a threshold (dash-dotted line).
Figure 18: Schematic set-up of the immuno-IR sensor with QCL.
Figure 19: Comparison of power density of different light sources. Also noise floors for different detectors are indicated. Figure 2 is taken from Weida et al. Proc. SPIE 7902, Imaging, Manipulation, and Analysis of Biomolecules, Cells, and Tissues IX, 79021C (February 10, 2011); doi:10.1117/12.873954.
Figure 20: Mid-IR absorbance spectra of showing the amide I absorbance band of Albumin with only one scan. The signal to noise ratio is sufficient to determine the absorbance maximum frequency.
Figure 21: Mid-IR absorbance spectra of showing the amide I absorbance band of Albumin with three scans.

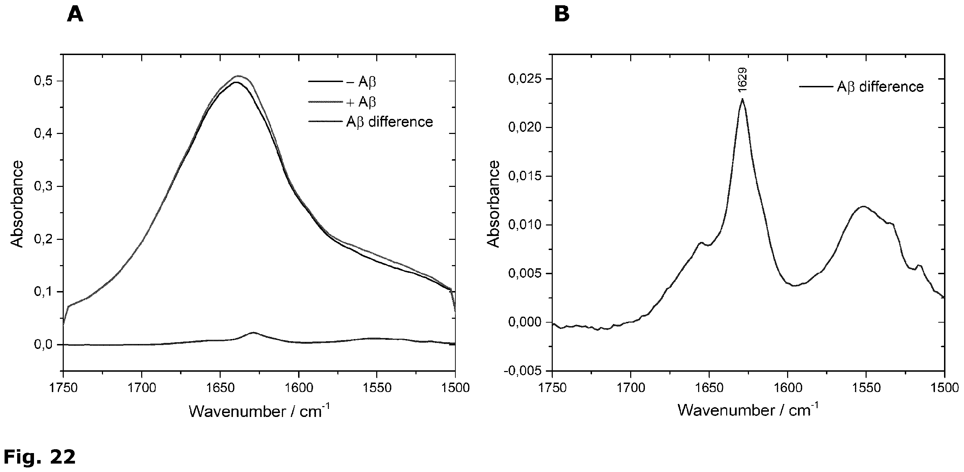

Figure 22: The absolute spectra measured with and without bound Aß measured with the QCL laser is shown in (A). The difference is shown in the lower part of (A) and in (B) with expanded scale. In order to detect the low concentrated Aβ fraction in liquid sample the difference has to be performed between a state where Aβ is bound to the sensor surface via specific antibodies and an unbound state. Thus, the much smaller Aβ absorbance spectrum can be detected without spectral contributions of water/buffer, antibodies, and other compounds attached to the ATR surface. Even the Aβ absorbance is much smaller than the background the S/N ration and the stability of the set-up allows by the difference technique to reveal the disease sensitive amide I band. The presented set-up provides sufficient signal to noise that reveals the amide I of the antibody bound biomarker. The amide I band frequency indicates the secondary structure distribution of Aß, which marks the disease.

### Detailed Description of the Invention

The invention is based on direct and intimate immobilization of receptors for the macromolecular substance to be analysed, i.e. antibodies on a germanium surface via silane or thiol chemistry with an optimized, simplified protocol. To analyze the liquid (e.g. blood or csf), it is fed to the sensor in a flow system. The macromolecular substance is immobilized by the antibody on the functionalized sensor surface.

The optical sensor element of aspects (1) and (1') of the invention is particularly suitable for infrared analysis and optionally further for the parallel analysis by another optical method including detection of fluorescence at different wavelengths. Furthermore, the sensor element is suitable for optical analysis of macromolecular substances including peptides and proteins, but also nucleotide-containing polymers such as DNA and RNA.

In a preferred embodiment of the optical sensor element of the invention the internal reflection element is a germanium crystal having a trapezoid or parallelogram shape, fiber or rod shaped geometry. It is preferred that the germanium crystal is a germanium monocrystal, while a trapezoid cut germanium monocrystal is particularly preferred.

It is further preferred that the germanium crystal allows for more than one passages of the infrared light through the reflection element, particularly preferred are more than five passages. For allowing the contact with the candidate biomarker protein in such multiple passages, the receptor for the biomarker protein is grafted to the appropriate number of surfaces of said internal germanium reflection element.

The silane and thiol linkers that are utilized for coupling the receptor and hence, the macromolecule to the internal germanium reflection element include homogenous silane and thiol linkers, mixtures of silane linkers and mixtures of thiol linkers. For allowing a tight and intimate linkage of the receptor/macromolecule short chained linkers, preferably linkers having a chain length of not more than 20 atoms or not more than 15 atoms, are utilized.

Such short chained linkers include silane linkers have one of the following formulas:

X₃Si-(CH₂)ₙ-Y-(CH₂)_{n'}-Z,

X₂R¹Si-(CH₂)ₙ-Y-(CH₂)_{n'}-Z

or

X(R¹)₂Si-(CH₂)ₙ-Y-(CH₂)_{n'}-Z,

and the thiol linkers have the following formula:

HS-(CH₂)ₙ-Y-(CH₂)_{n'}-Z,

wherein X at each occurrence is independently selected from halogen and C₁₋₆ alkoxy, n is an integers of 1 to 10, n' is an integer of 1 to 5; R¹ at each occurrence is independently selected from C₁₋₆ alkyl, Y is selected from a chemical bond, -O-, -CO-, -SO₂-, -NR²-, -S-, -SS-, -NR²CO-, -CONR²-, -NR²SO₂- and -SO₂NR²- (wherein R² is H or C₁₋₆ alkyl), and Z is an amine-reactive group including -CO₂H, -SO₃H and ester derivatives thereof.

The halogen within the present invention includes a fluorine, chlorine, bromine and iodine atom. C₁₋₆ alkyl and C₁₋₆ alkoxy includes straight, branched or cyclic alkyl or alkoxy groups having 1 to 6 carbon atoms that may be saturated or unsaturated. In case of cyclic alkyl and alkoxy groups, this refers to those having 3 to 6 carbon atoms. Suitable C₁₋₆ alkyl and C₁₋₆ alkoxy groups include, among others, methyl and methoxy, ethyl and ethoxy, n-propyl and n-propoxy, iso-propyl and iso-propoxy, cyclopropyl and cyclopropoxy, n-butyl and n-butoxy, tert-butyl and tert-butoxy, cyclobutyl and cyclobutoxy, n-pentyl and n-pentoxy, cyclopentyl and cycloppentoxy, n-hexyl and n-hexoxy, cyclohexyl and cyclohexoxy, and so on. The amine-reactive group Z includes all types of functional groups that are reactive with a free amino group. Among those, -CO₂H, -SO₃H and ester derivatives thereof (including active esters) are particularly preferred.

The -(CH₂)ₙ- and -(CH₂)_{n'}- structural elements in the above formulas may also contain one or more double and/or triple bonds and may be substituted with one or more halogen atoms such as fluorine.

In a preferred embodiment of the invention, X in the silane linkers is independently selected from C₁₋₆ alkoxy groups, preferably from methoxy and ethoxy groups, Y is - NHCO-, Z is -CO₂H or an ester derivative thereof, and n is an integer of 1 to 5 and n' is an integer of 1 to 3, preferably n is 3 and n' is 2.

In another embodiment, Y in the thiol linkers is a chemical bond, Z is -CO₂H or an ester derivative thereof, and n is an integer of 1 to 8 and n' is an integer of 1 to 5, preferably n is 8 and n' is 4. Particularly preferred is a 12-mercaptododecanoic acid NHS ester.

In another preferred embodiment of the optical sensor element at least one receptor for the macromolecular substance is a specific antibody. Furthermore it is preferred that the macromolecular substance is a protein that is characteristic for a protein misfolding disease such as, but not limited to, Alzheimer's disease (Aβ peptides and tau protein), Parkinson's disease ((alpha)-synuclein), Creutzfeldt-Jakob disease (prion protein), or chorea Huntington (huntingtin protein), preferably the macromolecule substance is an amyloidogenic peptide or a (poly-) peptide of health-status dependent, characteristic secondary structure composition.

The blocking substance not cross-reacting with the candidate biomarker protein includes casein, ethanolamine, L-lysine, polyethylene glycols, albumins, and derivatives thereof, and preferably is casein.

When the candidate biomarker protein is Aβ peptide, the antibody is an antibody specifically binding to the central epitope of the amyloid-beta peptide, such as antibody A8978 (Sigma Aldrich) and when the candidate biomarker protein is alpha-synuclein, the antibody is an antibody specifically binding to the alpha-synuclein peptide without conformational specificity, such as antibody 4B12 (Covance, BioLegend Inc.) or S5566 (Sigma Aldrich).

The device of aspect (2) of the invention has the sensor element of aspects (1) or (1') of the invention incorporated in a suitable IR cell (chamber). It may further include a light (IR) emitting element, a light (IR) detecting element and a data processing unit. For parallel detection by an additional optical method the device may further include light source and detector element for such additional optical method such as light source and detector elements for UV/Vis-fluorescence, at different wavelengths.

In the method of aspects (3) and (3') of the invention, the oxidization is performed by treatment with HzOz/oxalic acid. Further, in the method the silanization with the short silane linkers is preferably performed with a silane derivative having the following formulas:

X₃Si-(CH₂)ₙ-(CH₂)_{n'}-Y,

X₂(R¹)Si-(CH₂)ₙ-(CH₂)_{n'}-Y

or

X(R¹)(R²)Si-(CH₂)ₙ-(CH₂)_{n'}-Y,

wherein the variables are as defined above. It is particularly preferred that an ester derivative of the CO₂H or SO₃H moiety in the definition of Y be used, which can be a simple C₁₋₆ alkyl ester, but can also be an activated ester such as an N-hydroxysuccinimid ester or any other activated ester derivate. It is also preferred in the method that the receptor is an antibody. It is further preferred that the blocking substance is casein.

In the method of aspects (4) and (4') of the invention, the surface activation is performed by treatment with HF (49%). Further, in the method the thiolation with the short thiol linkers is preferably performed with thiol linkers having the following formula:

HS-(CH₂)ₙ-Y-(CH₂)_{n'}-Z,

wherein the variables are as defined above. It is particularly preferred that an ester derivative of the COzH or or SO₃H moiety in the definition of Y be used, which can be a simple C₁₋₆ alkyl ester, but can also be an activated ester such as an N-hydroxysuccinimid ester or any other activated ester derivate. It is also preferred in the method that the receptor is an antibody. It is further preferred that the blocking substance is casein.

In both aspects (3)/(3') and (4)/(4') the method of the optical sensor element is built up under room temperature without aggressive chemicals. Every single step can be assessed on the basis of the IR-spectra. This validation step is essential for the specific detection and accurate secondary structure determination of the analyte.

The method of aspects (6) and (6') of the invention comprises the steps of
(a) conducting, in an IR cell comprising the optical sensor element as defined herein before, a flux of potential macromolecular ligands for the receptor on the surface of said optical sensor;
(b) submitting an IR beam through said cell and obtaining an infrared spectrum therefrom; and
(c) analyzing the obtained infrared spectrum to determine the secondary structure, and optionally the quantity, of the macromolecular substance.

It may further include the step (d): analyzing the obtained infrared spectrum to classify the sample with statistical methods based on the secondary structure composition of the macromolecular substance.

In a preferred embodiment the method further comprises prior to step (a): installation of said optical sensor element in the IR cell. Additionally/alternatively the method may further comprise the step (e): regenerating of the surface of the optical element by application of a solution of free ligand for the receptor.

In a further preferred embodiment the spectrum obtained in step (b) has a sufficient signal to noise ratio to resolve the amide I band. This allows that step (c) preferably comprises the analysis of the shift of the amide I band maximum of the biomarker protein to determine the secondary structure of the candidate biomarker protein; and/or

In a further embodiment the step (c) of the method further comprises comparing the obtained infrared spectrum with a spectrum of the macromolecular ligand with known secondary structure and/or with known concentration.

In another embodiment, the method further comprises, parallel to the infrared analysis, detection by another optical method, including UV/Vis-fluorescence, at different wavelengths. Notably, a method is preferred that combines immuno-ATR-IR vibrational spectroscopy with parallel fluorescence spectroscopy.

The methods of aspects (6), (6') and (7) allow/ are suitable for determining macromolecules in bodily fluids, notably for directly determining candidate biomarker proteins in bodily fluids of mammalian (human, animal) origin, including cerebrospinal fluid, blood or serum, without pretreatment (i.e., without a separate preceding enrichment or purification step). The method is suitable for determination of the candidate biomarker protein in a separate (*in-vitro*) or an online (direct determination of the body fluid on the patient) fashion. In both cases, the method may further comprise the assessment of the disease progression.

The methods of aspects (6), (6') and (7) are particularly suitable for the determination of progression of Alzheimer's disease with amyloid-beta as candidate biomarker protein, wherein a shift of the amide I band maximum of the amyloid-beta peptide from 1647 cm⁻¹ to 1640 cm⁻¹, preferably with a threshold value of 1643 cm⁻¹ +/- 5 cm⁻¹, (or 1643 cm⁻¹ +/- 3 cm⁻¹, or 1643 cm⁻¹ +/- 1 cm⁻¹, or about 1643 cm⁻¹), is indicative for Alzheimer's disease. The amide I band frequency indicates the secondary structure distribution of Aβ. This structure distribution correlates with the Alzheimer's disease state. These methods are also particularly suitable for the determination of progression of Parkinson's disease with alpha-synuclein as candidate biomarker protein, wherein a down shift of the amide I band maximum of the alpha-synuclein peptide from 1646 cm⁻¹ to 1641 cm⁻¹, preferably with a threshold value of 1643 cm⁻¹ +/- 5 cm⁻¹ (or 1643 cm⁻¹ +/- 3 cm⁻¹, or 1643 cm⁻¹ +/-1 cm⁻¹, or about 1643 cm⁻¹), is indicative for the progression of Parkinson's disease. The amide I band frequency indicates the secondary structure distribution of Aβ. This structure distribution correlates with the Parkinson's disease state.

The invention of the present application provides for the specific immobilization of receptors for macromolecular substrates such as functional antibodies on the surface of an optical element in a direct and tight manner. In contrast to the above mentioned WO 02/056018 and EP-A-1806574, which disclose the chemical functionalization of the optical element with long chain silanes and carbohydrates in order to analyze the receptor immobilization, or the receptor interaction with ligands, the tight immobilization of the receptors for macromolecules such as highly specific antibody allows for the conformational analysis of a given macromolecule as a component of a complex fluid.

With the sensor of the invention the detection limit for the Aβ-peptide, a prime biomarker candidate for Alzheimer's disease, was two magnitudes lower as compared to the natural concentration in csf, and about one magnitude lower as compared to the natural concentration in blood. In the course of Alzheimer's disease, the Aß peptide conformation is changed. Conventional assays only include the concentration and the ratio of Aß-peptides with various chain lengths in the csf. With the sensor of the present invention, different Aß-conformations can be detected in real-time, and the measured absorbance presents an average signal of the present secondary structures. For Alzheimer patients, significant and specific changes in the conformational sensitive spectral region compared to control patients could be identified. Thereby, the sensitivity and practicability of the technique was shown. Electromagnetic radiation has to be coupled into the sensor element of the invention. The usable wavelength comprises Ultraviolet to Terahertz. For prototype development, the medium infrared (MIR) region was utilized. The antibody-bound substance absorbs radiation at specific wavelengths, generating an absorbance spectrum. The intensity of the absorbance signals allows for the quantitative interpretation of the substance concentration. The absorbance wavelength enables the direct, qualitative interpretation of e.g. secondary structure in case of proteins. In addition, the sensor element of the invention is designed for a parallel detection of at least two wavelength ranges with at least two distinct, but simultaneously applied spectroscopic methods, e.g. infrared absorbance and fluorescence measurements of the analyte.

The overall process can be largely automated. Therefore, the device and method of the invention is also operable for non-scientific personnel. The coupling of the method with classifying statistics for diagnostic purposes is also contemplated.

With the established sensor technique, defined substances can be analysed qualitatively and quantitatively directly within complex solutions, if antibodies for the desired substance are available. Therefore, a direct secondary structure analysis of proteins in untreated bodily fluids has become accessible. The specificity of the sensor is based on the specificity of the antibody. For basic research, the invention in particular enables the structural analyzes of proteins from solutions of low concentration.

By applying a bioinformatical classifier, several states of the attached substance can be differentiated automatically. The sensitivity and specificity of the discrimination has to be validated for each case.

The sensor opens the new field to search for new biomarkers with conformational classification. The use of our invention in clinical applications is in particular relevant, because bodily fluids can be analyzed directly after extraction. Only the predefined component is detected, and both the amount as well as its structure is analysed for diagnostic information.

A particular example is a neurodegenerative disease like Alzheimer's, which exhibits altered amounts and structures of the so far identified biomarker candidate molecules Aβ and tau in the csf. With the invention of the present application, both parameters are detected simultaneously.

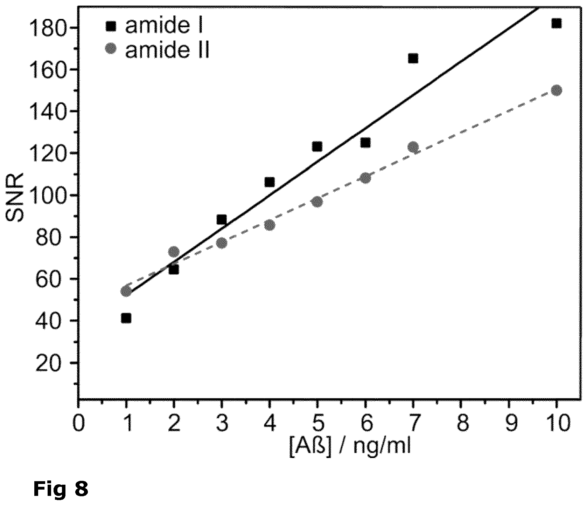

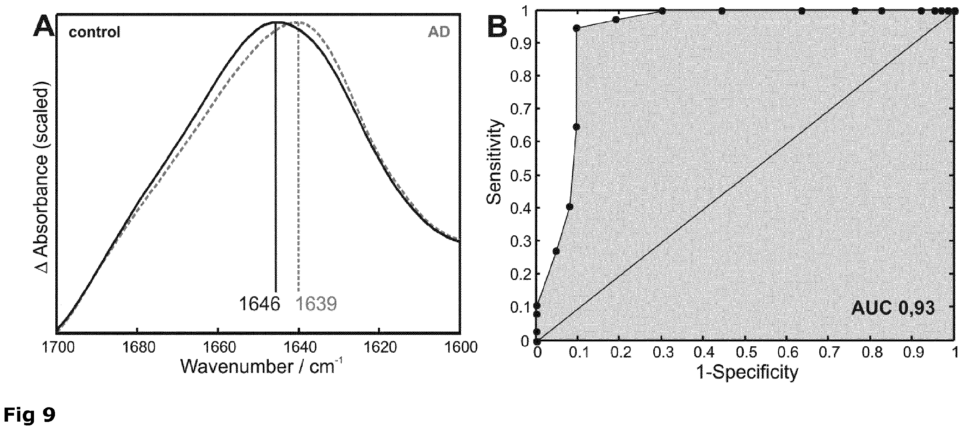

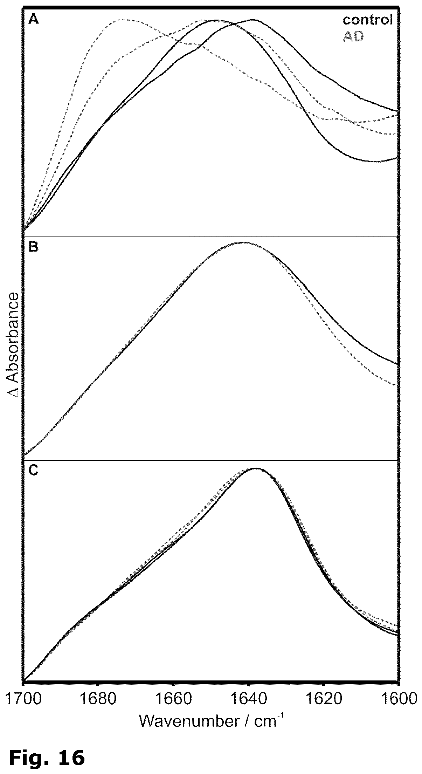

The invention provides for the secondary structure analysis of a protein amide I band of a specific protein within a complex fluid. The secondary structure is used as biomarker for the disease state. The structural sensitive frequency of the amide I in presence of the corresponding biomarker below a here defined threshold indicate the disease state. The threshold is described in Figs. 9, 10 and 11 for the Aβ peptide and in Fig. 16 for alpha-synuclein. The following detailed discussion is focussed on Aβ peptide for Alzheimer and alpha-synuclein for Parkinson. This threshold is the novel finding. In order to determine the threshold, an experimental setup which provides sufficient signal to noise ratio of the amide I band in presence of the body-fluid was invented. It is important to measure the secondary structure of the biomarker in the presence of the body-fluid, because it is very sensitive to the measuring conditions. For example the Aβ secondary structure varies between alpha helix and random coil depending on the measuring conditions. As example in the application the Aβ peptide is used. An excellent S/N of the amide I band at analyte concentrations below physiological level (Fig. 8) was obtained. Aβ peptides are concentrated at approximately 10-15 ng/ml in human cerebro spinal fluid (CSF). The demonstrated detection limit undercuts this value at least one order of magnitude.

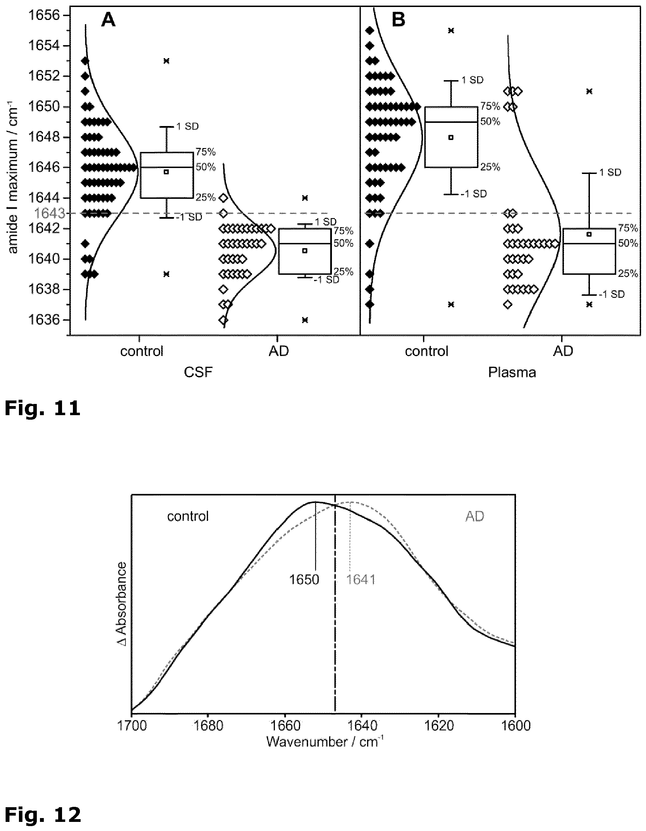

The intended protein is detected, as shown with the example Aβ peptide: capture of the synthetic Aβ peptide from defined, buffered solutions and from complex, conditioned cell culture medium were confirmed by the optional control analysis fluorescence (Fig. 11).

Based on the results of Examples 2 and 3, a threshold classifier with a value of 1638-1648 cm⁻¹ is a characteristic of the invention.

The preferred optical material of the invention is germanium. It is found that a so-called "BiaATR"-setup with only one single reflection geometry is of insufficient signal quality for the secondary structure analysis of Aβ peptides at physiological concentrations. Even if Aβ peptides were provided in >5fold excess in a pure and deuterated solution, a secondary structure analysis could not be performed on the low S/N spectra achieved [Kleiren et al. Spectroscopy 2010]. Therefore, the threshold as marker for the disease cannot be determined by this approach. A preferred embodiment of the invention features a multi-reflection crystal IRE of trapezoid shape. A parallelogram shape would be closely related and similarly possible. It appears crucial to exploit at least 5 internal reflections at the functionalised surface to achieve sufficient signal to noise ratio in order to determine the threshold.

A blocking step of the antibody-saturated surface is crucial for the intended amide I band analysis of the analyte. A detergent-free solution of a globular protein, unreactive with the analyte, but reactive with the silane or thiol linker, is used for chemical quenching / blocking of unspecific binding sites of the sensor element (see Examples 5 and 6).

The finding that a non-crossreacting blocking substance such as casein is required for conformational analysis, notably amide I band analysis is important for the present invention. An immunological blocking standard, albumin, is unsuitable for the specific Aβ peptide detection (see Example 7).

In immunological protocols, often dry milk powder is applied in buffered solutions. A buffered dry-milk solution is inapplicable for Aβ detection because it contains albumin. Detergents are not necessary for the sensor system. All examples have been performed with detergent-free solutions. Complications with the invention are not expected with common low concentrations of detergents as used in immunological protocols.

For the analysis of the Aβ peptide secondary structure in a sample, the antibody has to be sensitive (specific) for a central peptide epitope, but insensitive to the epitope structure the peptide (see Example 8).

The methods of aspects (6) and (7) of the invention are applicable for a variety of conformational diseases, also known as protein misfolding diseases, or proteopathies, which are caused by the misfolding of the following peptides/proteins: Amyloid-beta (Aβ) peptides and tau protein (Alzheimer's Disease (AD)); alpha-synuclein (Parkinson's Disease (PD)), prion protein (Creutzfeldt-Jakob disease (CJD), Bovine spongiform encephalopathy (BSE) commonly known as mad cow disease), huntingtin protein (chorea Huntington).

Specifically shown is the use for analysis of Aβ (Examples 1 to 8) and alpha-synuclein (Example 9).

The invention is further explained in the following non-limiting examples.

### Examples

### Material and methods

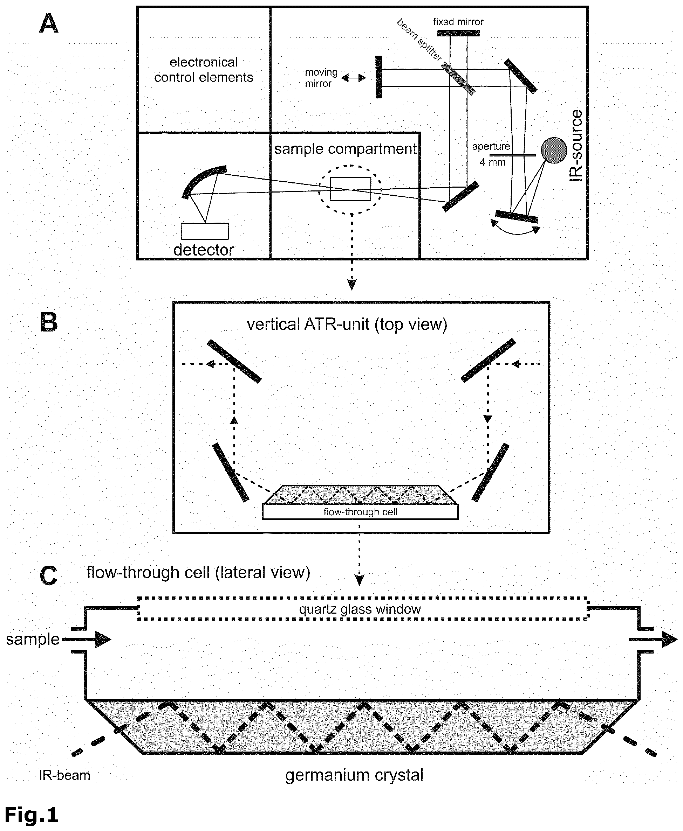

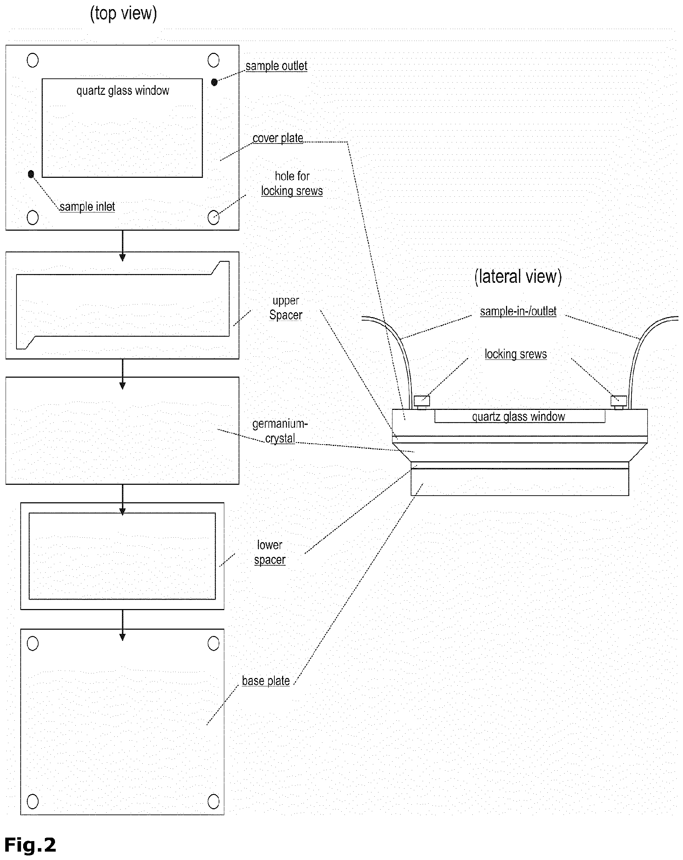

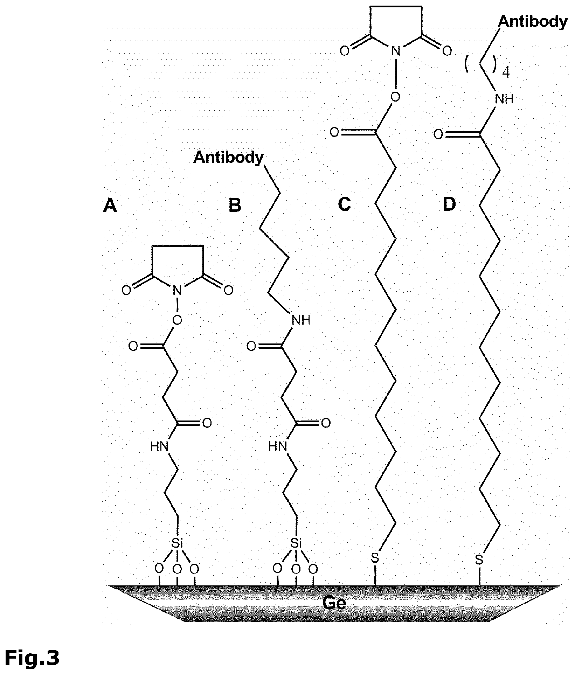

The invention can be used directly with an IR-spectrometer equipped with the commercially available sample compartment "GS11000 - 25 Reflection Variable Incidence Angle ATR" of Specac (Specac Ltd., Slough, England) (Fig. 1 A, optical path Fig. 1 B). The optical element, a germanium ATR-crystal (52 X 20 X 2 mm, Korth Kristalle GmbH, Altenholz (Kiel), Germany), was enclosed in an optimized bracket (Fig. 1 B, 2). Subsequently specified chemical modifications of the crystal surface generated the specific sensor-property (Fig. 3). If not mentioned otherwise, all chemicals were purchased from Sigma-Aldrich (Munich, Germany). Buffers and water were degased in the ultrasonic bath.

Sample set: The feasibility study first included 23 patients, 9 patients with a best possible confirmed Alzheimer diagnosis and 14 non-neurodegenerative controls. With continued recruiting, analyses were performed with csf samples of 37 AD and 63 control patients, and blood plasma samples of 35 AD and 61 control patients. The diagnosis of the patients is based on psychological reports, MRT-imaging data, results of csf and blood analysis, and psychometric test diagnostics. Based on availability, PET (positron emission tomography) or SPECT (single photon emission computed tomography) findings were considered. Additional reports results from course observations involving close relatives.

Sampling and pretreatment: Csf was drawn by lumbal puncture and aliquoted at the university hospital Essen, snap-frozen in liquid nitrogen, shipped and stored at -80 °C. Samples were not pretreated before the measurement, only thawed at 37 °C for 30 seconds and kept on ice until used.

Phosphate bufferd saline (PBS-buffer): 137 mM sodium chloride (NaCl), 2.7 mM potassium chloride (KCl), 12 mM total-phosphate (in the form of Na₂HPO₄ and NaH₂PO₄), pH 7.4.

Reaction-phosphate buffer: 50 mM Na₂HPO₄ / NaH₂PO₄, pH 8.0.

Casein blocking-solution: 200 mM sodium hydroxid (NaOH), 1 % (w/v) casein from bovine milk (powder), pH adjusted with H₃PO₄ to 7.4.

Silanization-solution: The silane used (N-(4,4,4-triethoxysilanebutyl)succinamic acid 2,5-dioxopyrrolidin-1-yl ester) was synthesized and characterized as described (J. Schartner et al., Journal of the American Chemical Society, 135(10):4079-4087 (2013)).

Antibody: The method was tested with two antibodies as capture molecules, 1E8 (Nanotools Antikörpertechnik GmbH, Teningen, Germany) and A8978 (lot no: 061M4773 Sigma Aldrich). 1E8 attaches to the N-terminal amino acids 1-11, A8978 attaches to the amino acids 13-28 of the amyloid-beta peptides. The fluorescence detection occurred through FITC-labeld 8G7 antibody (Nanotools), which recognizes the C-terminus of Aß₁₋₄₂ peptides. In addition conformation sensitive antibodies against oligomeric states (KW1) were utilized (Morgado et al., PNAS, 109(31): 12503-12508 (2012)) and fibrillar states of Aβ-peptides (B10) (G. Habicht et al., Proc. Natl. Acad. Sci. USA, 104(49):19232-19237 (2007)).

Preparation of the sensor surface with silanes: The Ge-IRE was bilaterally polished with 0.1 µm grained diamond grinding suspension for 5 min (Struers A/S, Ballerup, Denmark). The crystal was incubated three times in a hydrogen peroxide/ oxalic acid mixture (9:1) for 5 min, rinsed with water between every incubation step and dried with nitrogen gas. Furthermore the crystal was immediately installed with optimized silicone wavers in the flow-through-cell. The flow-rate was regulated at 1 ml/min by a peristaltic pump (IDEX Health&Science GmbH, Wertheim, Germany). The total-volume of the system amounted to 650 µl.

The sensor surface was incubated with 300 µM silane solution (Fig. 3) in 2-propanol for 60 min, unspecifically linked silane was rinsed with 2-propanol for 30 min. After media change to the reaction buffer, 25 µg/ml antibody solution was flushed over the activated silane surface until saturation, monitored by the immobilization kinetics of the amide II band of the antibody. Unspecifically bound antibody was rinsed with PBS-buffer until an equilibrium of the amide II absorbance was achieved. Free reaction sites of the sensor surface were saturated with casein blocking solution followed by rinsing with PBS buffer.

Preparation of the sensor surface with thiols: The Ge-IRE was prepared identically as described for silanization. The crystal was prepared as described by (S.M. Han et al., JACS, 123(10):2422-2425 (2001)). After HF treatment, the crystal was immediately immersed into an isopropanol solution containing 1 mM 12-mercaptododecanoic acid NHS ester. The monolayer was assembled after 24 h, the crystal was dried with N₂-gas and immediately installed into the ATR set up. Unbound thiols were removed by washing for 30 min with isopropanol. Further preparation was identical to the silanization protocol.

Performing the measurement: IR-measurements were performed on a Vertex 70V spectrometer (Bruker Optics GmbH, Ettlingen, Germany) with liquid nitrogen cooled mercury-cadmium-telluride (MCT) detector. Double-sided interferograms were recorded in forward-backward interferometer movement at a 60 kHz data rate with a spectral resolution of 2 cm⁻¹, Blackman-Harris-3-Term-apodisation, Mertz-phase correction and 4 times zero filling. Reference spectra were recorded as an average of 1000, sample spectra of 200 interferograms. Recording reference single channel spectra of the blank sensor, sensor with 2-propanol, the silanized surface, the buffers, antibody or casein coated surface in equilibrium states enabled high sensitivity difference spectroscopy based on Lambert-Beer law (E=-log(I/I₀). The absorbance of the state change is the negative decadic logarithm of the intensity relation before and after the change. 50 µl csf were added to the PBS-buffered system in a circulating flow for the secondary structure analysis of the Aß-peptide fraction. After the binding equilibrium was achieved, unbound material was rinsed with PBS-buffer from the system until no spectral changes were observed. Thus, the Aß absorbance spectrum was calculated from the difference between this state and the casein blocked, PBS rinsed sensor surface.

Pretreatment of the spectra: Spectral traces of atmospheric water vapor were removed by scaled subtraction of a reference spectrum. High frequency noise with a full width at half height (FWHH) of less than four wavenumbers was removed through a Fourier low pass filter. Spectra were baseline corrected as described (J. Ollesch et al., The Analyst, 138(14):4092 (2013)), and normalized to the same amide I signal intensity between 1730 and 1620 cm⁻¹ before classification.

Classification: In order to reduce the dimensionality of spectral data, the position of the amide I maxima of the average spectra of both patients groups was chosen as classification relevant data points. The classification of the data resulted from a linear discriminant analysis (LDA), by matlab programming environment, version 2012a. The program intern function ('classify') was used. All calculations were done on an office PC with Intel Core2Quad CPU Q9650@3.0 GHz, 8 GB RAM (Dell Optiplex 780).

### Example 1: Analysis of the Amide I band in Aß with Silane-coupled Linkers

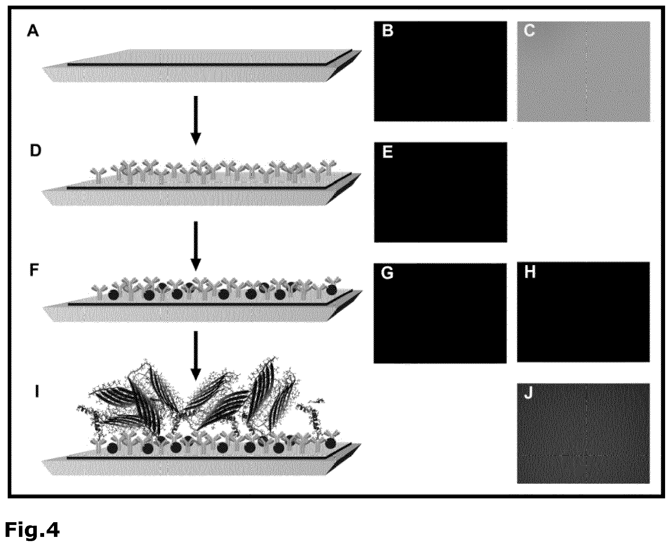

The specific sensitivity of the established sensor setup (Fig. 1, 2) is defined by the antibody. By using fluorescence microscopy it was possible to determine fluorescence only if the FITC coupled antibody 8G7 was attached onto the surface. The control did not reveal any fluoresecence (Fig. 4). The antibody 8G7-FITC was attached covalently to the amine-reactive silane surface (Fig. 3 A, B) and could not be removed by washing with buffer (Fig. 4 C). Another tested antibody 1E8 did not show any fluorescence (Fig. 4 E). After blocking open, nonspecific binding sites with casein, no further binding of the 8G7 was observed, which shows that 8G7 neither binds to casein nor to 1E8. If looking on 8G7-FITC as a protein in general, this experiment shows the silane surface being shielded from unspecific interaction with the contained proteins in the sample.

Only after the incubation with the Aß₁₋₄₂ peptide, a fluorescence signal was detected from the subsequent 8G7-FITC labeled surface (Fig. 4 J), which proves the successful immobilization of Aβ. Furthermore, it was shown that the designed sensor allows parallel experiments with different optical techniques.

The conformational sensitivity of the analyzed amide I band was proven with monomeric, oligomeric and fibrillized Aß₁₋₄₂ peptide (Fig. 5 A). The fibrillar and oligomer states differ strongly from non-aggregated peptide, which can be seen by the higher amount of β-sheets. This can be revealed by a shift of the amide-I-maximum towards 1624 cm⁻¹ and 1630 cm⁻¹. The high-frequency component at 1665 cm⁻¹ is fibril characteristic. The oligomerized Aβ-peptide is discussed as a toxic intermediate in the formation of amyloid plaques in Alzheimer patients (I. Benilova et al., Nature Neuroscience, 15(3):349-357 (2012)). Oligomers have a different β-sheet structure compared to monomers and fibrils. A possible explanation is the higher amount of antiparallel sheets (Cerf et al., The Biochemical Journal, 421(3):415-423 (2009); Yu et al., Biochemistry, 48(9):1870-1877 (2009); Laganowsky et al., Science, 335(6073):1228-1231 (2012)) (Fig. 5 A, shoulder in the green band at about 1682 cm⁻¹). This implies a different amide I band for monomers and fibrils.

With the conformationally sensitive antibodies B10 (fibrils) and KW1 (oligomers), we were able to detect both corresponding Aβ-peptide fractions within the same natural human csf of a control patient (Fig. 5 B, C). In comparison with a synthetic solution of isolated Aβ peptides, in which only the secondary structure of the isolated peptide in defined conformation is revealed, the data from Fig. 5 B and C presents the expected secondary structure compositions as present in the natural body fluid.

Therefore, conformationally sensitive antibodies are not suitable for the detection of disease related structural changes. These antibodies specifically detect only the desired conformation, but the important feature is the proportionate composition of monomers, oligomers and amyloid fibrils. With the conformationally independent antibody A8978, the detected amide I band resembled the structural composition of the Aβ-peptide fraction quantitatively. It is likely, that this causes the high specificity of our sensor technique to discriminate patients (Fig. 5 D, E).

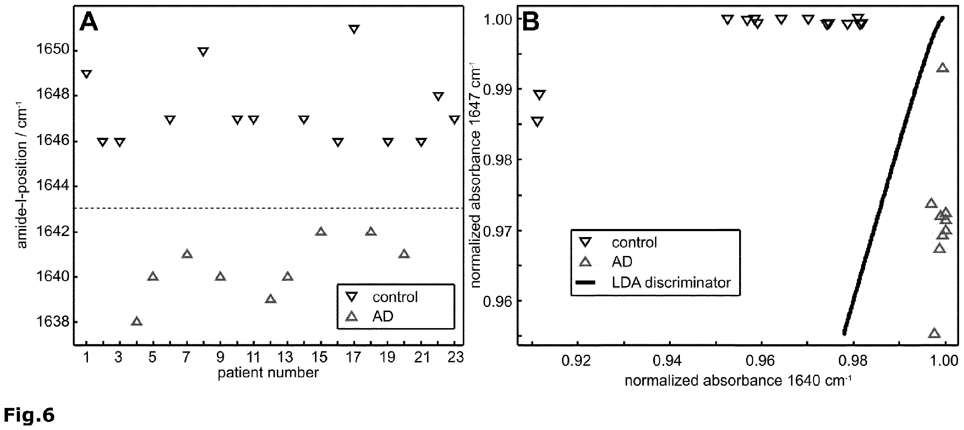

The discrimination of control- from Alzheimer patients based on the amide I maximum position is possible with 100 % accuracy (Fig. 6 A).

An alternative classifier, an LDA based on the amide I intensity at 1647 and 1640 cm⁻¹, results in 97±6 % average accuracy with 94±11 % sensitivity and 100±0 % specificity, based on a 1000 fold repeated Monte Carlo cross validation leaving one third of data out for classifier validation (Fig. 6 B).

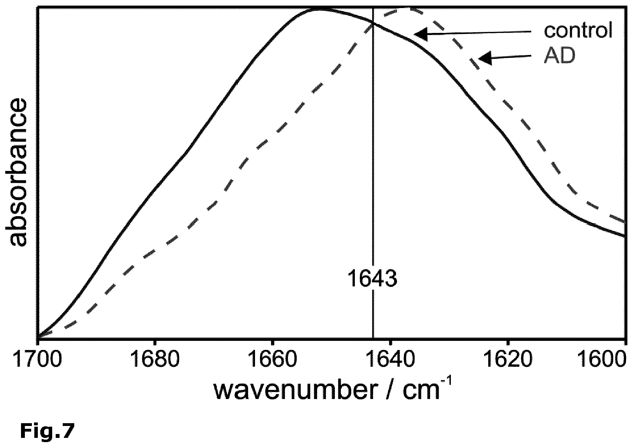

With the antibody linked covalently via 12-mercaptododecanoic acid NHS ester as thiol linker on the germanium IRE (Fig. 3 C, D), an identical discrimination of AD from control patients based on the secondary structure of the EDTA stabilized blood plasma Aß-peptide fraction was achieved (Fig. 7).

Example 2: Analysis of Aß peptide structure in CSF of Alzheimer's Disease (AD) patients and a control group, extended data set of 37 AD and 63 controls.

The original exemplary analysis of 20 samples was extended to 100 patients. The average conformation of Aß peptides, as present in CSF, exhibited a higher amide I band frequency in the control than the AD group (Fig. 9A). This indicated a predominant alpha-helical fold in the control, whereas the AD group already exhibits an enriched beta-sheet component. Using the amide I band frequency as indicator, 1643 cm⁻¹ so far represents the optimum threshold for discrimination of the classes with an accuracy of 92 %, a sensitivity of 95 %, and specificity of 90 %. The according receiver operator characteristic (ROC) curve depicts an area under curve (AUC) of 0.93 (Fig. 9B).

Example 3: application for the analysis of Aß peptide structure in EDTA-stabilized blood plasma of 35 AD and 61 control patients.

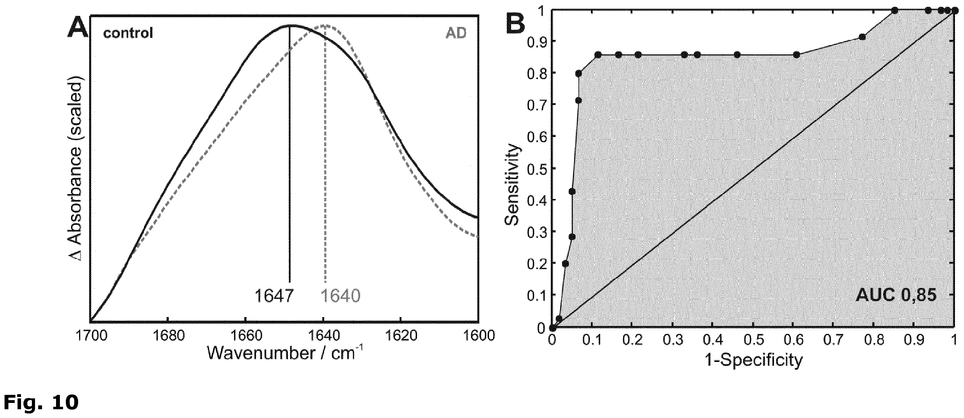

As with CSF, the average conformation of Aß peptides detected in blood plasma exhibited a higher amide I band frequency in the control than the AD group (Fig. 10A). This indicated the same disease influence on the blood-borne Aß peptide fraction. Again, 1643 cm⁻¹ represents the optimum frequency threshold for discrimination of the classes with an accuracy of 89 %, a sensitivity of 80 %, and specificity of 93 %. The according ROC curve depicts an AUC of 0.85 (Fig. 10B).

In Examples 2 and 3 CSF samples of 37 AD and 63 control patients, and blood plasma samples of 35 AD and 61 control patients were analysed.

The according histogram and box plots confirmed the findings with well differentiated maxima of the distributions (Fig. 11). All classes were well approximated with a Gaussian normal distribution. The average band positions of the CSF control and AD classes did not overlap with ±1 standard deviation. A two-sided t-test indicated a significant class difference with p<0.001 for both sample groups, CSF and plasma. The maximum amide I band positions of the Aß peptide fractions were well separable by a simple classifying threshold: band maxima below 1643 cm⁻¹ were assigned to the AD class, equal or above 1643 cm⁻¹ as control patients. This threshold represents the classifier with optimum accuracy of 92 % for CSF, and 89 % for blood plasma samples.

Based on t-test statistics at 99.9 % confidence level, a generalized classifying threshold is expectable in a range of 1638-1648 cm⁻¹.

Example 4: Aβ peptide captured from AD and control patient CSF via a thiol linker.

The germanium IRE of the setup was polished, cleaned with acetone, incubated in HF (10 min 40 % at room temperature), washed with distilled water, blown dry in N₂, modified over night with a thiol linker (12-mercapto-undecanoic-acid-NHS-ester, Fig. 3C, 3D), buffer-rinsed, functionalized with antibody A8978, and desensitised with casein. The Aβ fraction of CSF samples of one AD and one control patient exhibited identical discriminative features as detected with silane linkers, the classifying threshold of 1643 cm⁻¹ proved valid (Fig. 12).

Example 5: An unblocked sensor element is receptive for Aβ peptides and two selected blood/CSF components.

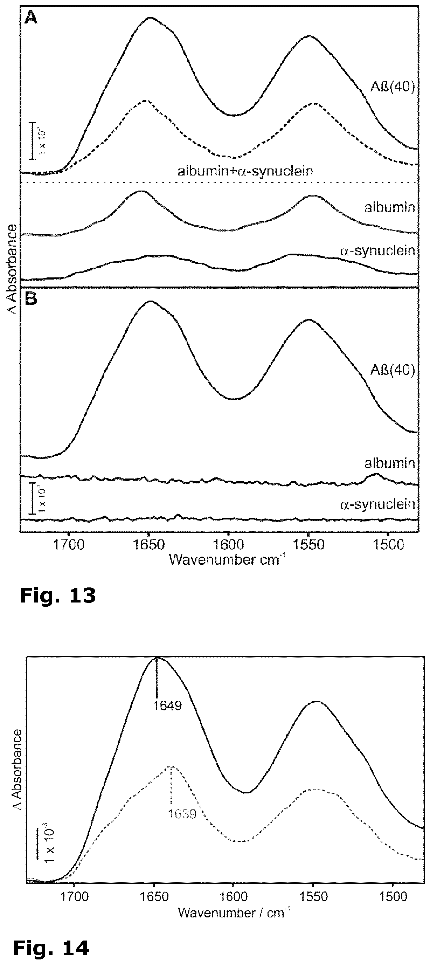

Three sensor elements were polished, silanized, and saturated with 1E8 antibody against Aβ peptides. Two were used after rinsing out unspecifically bound antibody molecules, one was blocked with casein solution and rinsed. On one unblocked sensor, alpha-synuclein was incubated at 20 ng/ml concentration, rinsed, and Aß₁₋₄₀ peptide was incubated at 15 ng/ml concentration, and rinsed. The other unblocked sensor was incubated with albumin at 25 µg/ml concentration, rinsed, and Aß₁₋₄₀ peptide was incubated at 15 ng/ml concentration, and rinsed. The sensor elements were receptive for alpha-synuclein and albumin at concentrations which are expectable in bodily fluids (Fig. 13A). The binding was unspecific, because the binding sites for Aβ peptides were free in either case, a regular Aβ peptide signal was recorded.

The blocked sensor was incubated with 20 ng/ml alpha-synuclein solution and rinsed, without observable binding to the sensor surface (Fig. 13B). Consecutively, the sensor was incubated with 25 µg/ml albumin and rinsed, again without observable albumin signal. Only the incubation with 15 ng/ml Aß₁₋₄₀ peptide solution resulted in a regular signal after rinsing (Fig. 13B).

Example 6: The sensor without blocking step is inapplicable for diagnostics.

Two sensor elements were prepared: one with, and one without casein blocking step. CSF aliquots of an AD patient were incubated on the sensors for the Aβ amide I band analysis. The amide I band intensities recorded at the unblocked surface exhibited a far higher, thus more unspecific binding (Fig. 14). Without blocking, the band position no longer correlated with the disease state: the unblocked sensor readout indicated a predominant alpha helical conformation at 1649 cm⁻¹, whereas the blocked sensor readout indicated beta-sheet enrichment with the Aβ maximum amide I frequency at 1639 cm⁻¹. A higher maximum frequency is thus attributable to the unspecific detection of the predominantly helical protein background in CSF.

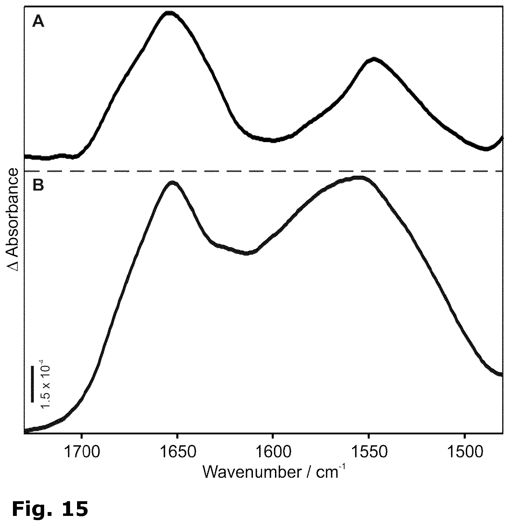

Example 7: With the sensor element prepared by silanisation, antibody functionalization, blocking with casein, incubation with conditioned cell culture medium, and rinsing of unspecifically bound proteins, a regular amide band pattern was observed: the amide II band was less intense than the amide I band (Fig. 15A). Fluorescence control analysis confirmed Aβ peptide binding (Fig. 4).

Contrastingly, the amide band pattern recorded with another aliquot of the same sample exhibited a higher amide II intensity, when albumin instead of casein was used for blocking. The overall amide I intensity was increased by approximately 100 %. Thus, albumin obviously provided additional unspecific binding sites for not further definable proteins and other substances featuring an absorbance between 1600-1500 cm⁻¹ (Fig. 15B).

Example 8: 1E8 and antibodies specific for oligomers and fibrils applied for CSF analysis.

The exemplarily demonstrated antibodies 1E8 and A8978 sense both monomerised and fibrillised Aß peptides (Fig. 6A). For diagnostic purposes, the enhanced specificity of A8978 antibody sensing a central Aβ epitope proved advantageous over the 1E8 antibody, when a complex body fluid was analysed. 1E8 is known to cross-react with sAPPalpha (N-terminal fragment of amyloid precursor protein (APP) after alpha-secretase processing) due to its N-terminal Aβ epitope. A disease-related conformation of sAPPalpha has not been reported, and the overlaying signal hinders a clear-cut sensor readout (Fig. 16A). For a stringent separation of AD from control Aβ peptide conformations, the enhanced specificity of a central epitope antibody as A8978 is required (Figs. 9, 10 and 11).

Using conformation-specific antibodies against oligomers (Fig. 16B) or fibrils (Fig. 16C) did not exhibit disease specific sample features. Both conformations were present in AD and control patient CSF samples. Therefore, the intended diagnostic system has to be prepared with antibodies that are unspecific for the epitope's conformation.

Example 9: Application of the sensor for alpha-Synuclein analysis as present in blood serum with regard to Parkinson's Disease (PD).

The sensor element was typically prepared, polished and silanized. For alpha-synuclein specific functionalization, 4B12 monoclonal antibody (Covance, BioLegend Inc.) was immobilized on the sensor, followed by casein blocking. The recorded spectra of alpha-synuclein as present in blood serum exhibited a clearly downshifted amide I band maximum frequency at 1641 cm⁻¹ in the PD sample as compared to 1646 cm⁻¹ recorded of the control patient sample. Thereby, the applicability of the sensor for label-free PD diagnostics on blood samples is shown (Fig. 17).

### Example 10:

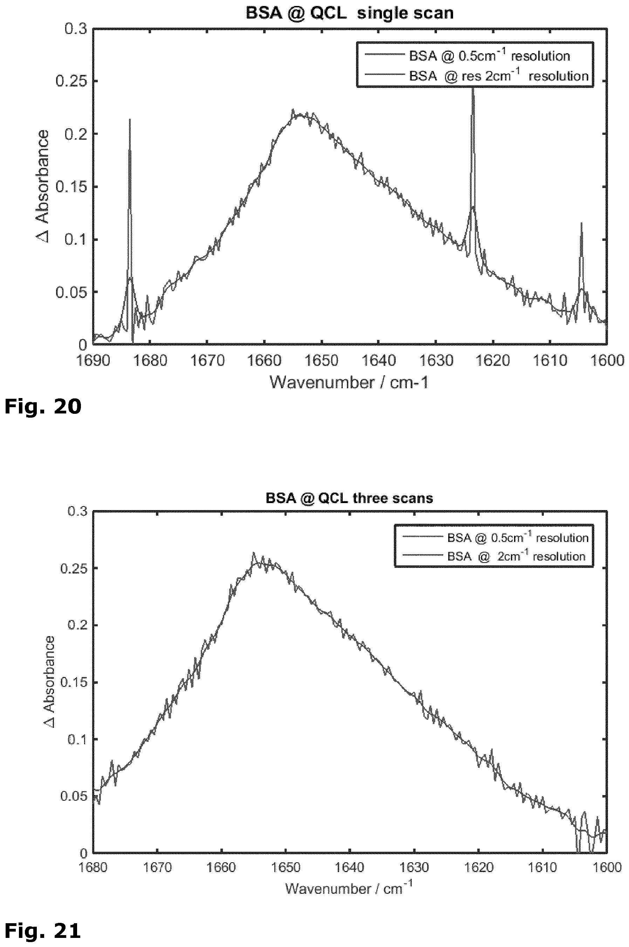

Protein amide 1 bands can be measured in an sensor element by activation with a tunable quantum cascade laser as shown by Figs 20 and 21.

### Example 11

The Aβ 1-42 spectrum measured with a tuneable QCL set-up as schematically illustrated in Figure 18 is shown in Figure 22. The set-up is based on a a tunable Quantum Cascade laser, which monitors the IR spectrum between 1750 and 1500 cm⁻¹ and an IR-detector (daylight solutions), which operates at room temperature. The used ATR cell is described in Figure 1 and 2. The ATR surface is prepared as described in detail for the FTIR measurements. It is silanized to covalently bind an capture-antibody and coated with casein to prevent unspecific binding. First a reference spectrum is taken, then Aβ 1-42 was added to the PBS-buffered system in a circulating flow. After the binding equilibrium was achieved, unbound material was rinsed with PBS-buffer from the system until no more spectral changes were observed. Then, a second absorbance spectrum with bound Aß was taken. Comparison of the two spectra without and with bound Aβ show a small deviation, but both spectra are dominated by the compounds bound to the surface of the ATR cell, especially the strong OH bending vibration of water and by the antibody spectrum. The shift of the of Aβ amide I band cannot be observed at this scale. The absorbance of the Aβ amide I band is about 2 orders of magnitude smaller than the background absorbance of other surface bound compounds, especially the capture antibodies and water/buffer. In order to reveal the amide I band of the bound Aβ protein only, which indicates the disease, one has to perform a difference-spectrum between the spectra taken before and after Aβ binding. Thereby, the background absorbance is eliminated. On a largely expanded scale the Aß spectrum can be seen. Figure 22 shows such difference spectrum. In order to perform such difference spectra at which the much larger background absorbance is eliminated, the set-up has to be very stable and has to provide an excellent signal to noise ratio to subtract the much larger background. Only the difference-spectroscopy provide the desired sensitivity to reveal the amide I band of the Aβ biomarker not the absolute IR absorbance spectra, which are usually measured. Figure 22 shows that the QCL based set-up provides a sufficient S/N ratio that a difference spectrum reveals only the Aβ spectrum without water and antibody contributions. The QCL set-up is much more compact than the FTIR set-up due to the much smaller coherent beam of the QCL as compared to the Globar used in FTIR. Therefor it will need about a factor of 10 less sample material. In additions it does not need liquid nitrogen cooling and can be used in any environment.

## Claims

1. An infrared detection system for the direct difference spectroscopical analysis of the quantity and secondary structure of a candidate biomarker protein undergoing conformational transitions associated with disease progression, said detection system comprising:
(a) a tunable quantum-cascade laser as the IR source, and
(b) an IR cell with an infrared sensor element that comprises a germanium internal reflection element being of trapezoid or parallelogram shape, which allows for more than one passages of the infrared light through the reflection element, and being transparent in the infrared with sufficient signal to noise ratio to detect the amide I band, and at least one receptor for the biomarker protein being an antibody capable of specific and conformationally independent binding to the candidate biomarker protein and being directly grafted to at least one surface of said internal germanium reflection element by silanization with short silane linkers or by thiolation with short thiol linkers, reacting freely accessible amine groups of said at least one receptor with amine-reactive groups on the short silane/thiol linkers, and blocking remaining amine-reactive groups on the short silane/thiol linkers with a blocking substance not cross-reacting with the candidate biomarker protein.

2. The infrared detection system of claim 1, wherein
(i) the sensor element is further suitable for the parallel analysis by another optical method including detection of fluorescence at different wavelengths; and/or
(ii) the internal reflection element is a germanium monocrystal, preferably is a trapezoid cut germanium monocrystal.

3. The infrared detection system of claim 1 or 2, wherein the silane and thiol linkers include homogenous silane and thiol linkers, mixtures of silane linkers and mixtures of thiol linkers, and have a chain length of not more than 20 atoms or not more than 15 atoms, preferably
the silane linkers have one of the following formulas:
X₃Si-(CH₂)ₙ-Y-(CH₂)_{n'}-Z,
X₂R¹Si-(CH₂)ₙ-Y-(CH₂)_{n'}-Z
or
X(R¹)₂Si-(CH₂)ₙ-Y-(CH₂)_{n'}-Z,
and the thiol linkers have the following formula:
HS-(CH₂)ₙ-Y-(CH₂)_{n'}-Z,
wherein X at each occurrence is independently selected from halogen and C₁₋₆ alkoxy, n is an integers of 1 to 10, n' is an integer of 1 to 5; R¹ at each occurrence is independently selected from C₁₋₆ alkyl, Y is selected from a chemical bond, -O-, -CO-, - SO₂-, -NR²-, -S-, -SS-, -NR²CO-, -CONR²- , -NR²SO₂- and -SO₂NR²- (wherein R² is H or C₁₋₆ alkyl), and Z is an amine-reactive group including -CO₂H, -SO₃H and ester derivatives thereof.

4. The infrared detection system of claim 3, wherein
(i) X in the silane linkers is independently selected from C₁₋₆ alkoxy-groups, preferably from methoxy and ethoxy groups, Y is -NHCO-, Z is -CO₂H or an ester derivative thereof, and n is an integer of 1 to 5 and n' is an integer of 1 to 3, preferably n is 3 and n' is 2; or
(ii) Y in the thiol linkers is a chemical bond, Z is -CO₂H or an ester derivative thereof, and n is an integer of 1 to 8 and n' is an integer of 1 to 5, preferably n is 8 and n' is 4.

5. The infrared detection system of any one of claims 1 to 4, wherein
(i) the candidate biomarker protein undergoing conformational transitions associated with disease progression is an amyloidogenic peptide or a (poly-) peptide of health-status dependent, characteristic secondary structure composition, including amyloid-beta peptides and tau protein (associated with Alzheimer's disease), alpha-synuclein (associated with Parkinson's disease), prion protein (associated with Creutzfeldt-Jakob disease), or huntingtin protein (associated with chorea Huntington); and/or
(ii) the blocking substance not cross-reacting with the candidate biomarker protein is selected from casein, ethanolamine, L-lysine, polyethylene glycols, albumins and derivatives thereof; and/or
(iii) the candidate biomarker protein is amyloid-beta peptide and the antibody is an antibody specifically binding to the central epitope of the amyloid-beta peptide, including antibody A8978; or
(iv) the candidate biomarker protein is alpha-synuclein and the antibody is an antibody specifically binding the alpha-synuclein peptide without conformational specificity, including antibody 4B12 or S5566.

6. The detection system of any one of claims 1 to 5, said system further comprising an IR-detector.

7. Use of the infrared detection system of any one of claims 1 to 6 for determining the secondary structure, and optionally the quantity, of a candidate biomarker protein undergoing conformational transitions associated with disease progression in a complex fluid including bodily fluids by difference spectroscopy.

8. A method for determining the secondary structure, and optionally the quantity, of a candidate biomarker protein undergoing conformational transitions associated with disease progression in a complex fluid by difference spectroscopy, comprising the steps:
(a) conducting, in the IR cell comprising the infrared sensor element of claims 1 to 6, a flux of potential candidate biomarker proteins for the receptor on the surface of said infrared sensor;
(b) submitting an IR beam through said cell with the tunable quantum-cascade laser and obtaining an infrared spectrum therefrom; and
(c) analyzing the obtained infrared spectrum to determine the secondary structure, and optionally the quantity, of the candidate biomarker protein by comparing the obtained infrared spectrum with a spectrum of the candidate biomarker protein with known secondary structure, and optionally with a known concentration thereof.

9. The method of claim 8, wherein
(i) the method further comprises the step (d): analyzing the obtained infrared spectrum to classify the sample with statistical methods based on the secondary structure composition of the candidate biomarker protein; and/or
(ii) the method further comprises the step (e): regenerating of the surface of the infrared element by application of a solution of free ligand for the receptor; and/or
(iii) the spectrum obtained in step (b) has a sufficient signal to noise ratio to resolve the amide I band; and/or
(iv) step (c) comprises analyzing the shift of the amide I band maximum of the biomarker protein to determine the secondary structure of the candidate biomarker protein; and/or
(v) the method further comprises, parallel to the infrared analysis, detection by another optical method, including UV/Vis-fluorescence, at different wavelengths; and/or
(vi) the method combines immuno-ATR-IR vibrational spectroscopy with parallel fluorescence spectroscopy; and/or
(vii) the method is suitable for directly determining candidate biomarker proteins in bodily fluids, including cerebrospinal fluid, blood or serum, without pretreatment; and/or
(viii) the method is suitable for separate (*in-vitro*) or online determination of the candidate biomarker protein.

10. The method of claim 8 or 9 which is for the determination of progression of a disease, in which a conformational transitions of a candidate biomarker protein is associated with disease progression, wherein a shift of the amide I band maximum of the biomarker protein is a classifier indicative for the progression of the disease, preferably a threshold classifier with a value of 1638-1648 cm⁻¹ is a classifier indicative for the progression of the disease.

11. The method of any one of claims 8 to 10, which is
(i) for the determination of progression of Alzheimer's disease with amyloid-beta as candidate biomarker protein, wherein a downshift of the amide I band maximum of the amyloid-beta peptide, preferably with a threshold value within 1638-1648 cm⁻¹ is indicative for progression of Alzheimer's disease; or
(ii) for the determination of progression of Parkinson's disease with alpha-synuclein as candidate biomarker protein, wherein a down shift of the amide I band maximum of the alpha-synuclein peptide, preferably with a threshold value within 1638-1648 cm⁻¹ is indicative for the progression of Parkinson's disease.

## Patentansprüche

1. Infrarot-Nachweissystem für die direkte Differenzspektroskopie-Analyse der Quantität und der Sekundärstruktur eines Biomarker-Kandidatenproteins, das Konformationsübergänge durchläuft, die mit dem Fortschreiten einer Krankheit verbunden sind, wobei das Nachweissystem umfasst:
(a) einen abstimmbaren Quantenkaskadenlaser als IR-Quelle, und
(b) eine IR-Zelle mit einem Infrarotsensorelement, das ein Germanium-Innenreflexionselement in Form eines Trapezes oder Parallelogramms umfasst, das mehr als einen Durchgang des Infrarotlichts durch das Reflexionselement ermöglicht, und das im Infrarot Bereich mit einem ausreichenden Signal-Rausch-Verhältnis transparent ist, um die Amid-I-Bande nachzuweisen, und mindestens ein Rezeptor für das Biomarkerprotein ein Antikörper ist, der zur spezifischen und konformationsunabhängigen Bindung an das Kandidaten-Biomarkerprotein fähig ist und direkt auf mindestens eine Oberfläche des internen Germanium-Reflexionselements durch Silanisierung mit kurzen Silan-Linkern oder durch Thiolierung mit kurzen Thiol-Linkern gegrafted ist, Reagieren von frei zugänglichen Amingruppen des mindestens einen Rezeptors mit aminreaktiven Gruppen an den kurzen Silan/Thiol-Linkern und Blockieren der verbleibenden aminreaktiven Gruppen an den kurzen Silan/Thiol-Linkern mit einer blockierenden Substanz, die nicht mit dem Kandidaten-Biomarkerprotein kreuzreagiert.

2. Das Infrarot-Detektionssystem nach Anspruch 1, wobei
(i) das Sensorelement ferner für die parallele Analyse durch ein anderes optisches Verfahren einschließlich der Detektion von Fluoreszenz bei verschiedenen Wellenlängen geeignet ist; und/oder
(ii) das interne Reflexionselement ein Germanium-Monokristall ist, vorzugsweise ein trapezförmig geschnittener Germanium-Monokristall.

3. Infrarot-Detektionssystem nach Anspruch 1 oder 2, wobei die Silan- und Thiol-Linker homogene Silan- und Thiol-Linker, Mischungen von Silan-Linkern und Mischungen von Thiol-Linkern umfassen und eine Kettenlänge von nicht mehr als 20 Atomen oder nicht mehr als 15 Atomen aufweisen, vorzugsweise
Wobei die Silanlinker eine der folgenden Formeln haben:
X₃Si-(CH₂)ₙ-Y-(CH₂)_{n'}-Z,
X₂R¹Si-(CH₂)ₙ-Y-(CH₂)_{n'}-Z
or
X(R¹)₂Si-(CH₂)ₙ-Y-(CH₂)_{n'}-Z,
Und die Thiol-Linker die folgende Formel haben:
HS-(CH₂)ₙ-Y-(CH₂)_{n'}-Z,
wobei X bei jedem Auftreten unabhängig ausgewählt ist aus Halogen und C₁₋₆ Alkoxy, n eine ganze Zahl von 1 bis 10 ist, n' eine ganze Zahl von 1 bis 5 ist; R¹ bei jedem Auftreten unabhängig ausgewählt ist aus C₁₋₆ Alkyl, Y ausgewählt ist aus einer chemischen Bindung, -O-, -CO-, -SO₂-, -NR²-, -S-, -SS-, -NR²CO-, -CONR²-, -NR²SO₂-and -SO₂NR²- (worin R² H oder C₁₋₆ Alkyl ist), und Z eine aminreaktive Gruppe ist, einschließlich -CO₂H, -SO₃H und Esterderivate davon.

4. Das Infrarot-Detektionssystem nach Anspruch 3, wobei
(i) X in den Silan-Linkern unabhängig ausgewählt ist aus C₁₋₆ Alkoxy-Gruppen, vorzugsweise aus Methoxy- und Ethoxy-Gruppen, Y -NHCO- ist, Z -CO₂H oder ein Ester-Derivat davon ist, und n eine ganze Zahl von 1 bis 5 ist und n' eine ganze Zahl von 1 bis 3 ist, vorzugsweise n 3 ist und n' 2 ist; oder
(ii) Y in den Thiol-Linkern eine chemische Bindung ist, Z -CO2H oder ein Esterderivat davon ist, und n eine ganze Zahl von 1 bis 8 ist und n' eine ganze Zahl von 1 bis 5 ist, vorzugsweise ist n 8 und n' ist 4.

5. Das Infrarot-Detektionssystem nach einem der Ansprüche 1 bis 4, wobei
(i) das Kandidaten-Biomarkerprotein, das Konformationsübergänge durchläuft, die mit dem Fortschreiten der Krankheit assoziiert sind, ein amyloidogenes Peptid oder ein (Poly-)Peptid mit einer vom Gesundheitszustand abhängigen, charakteristischen Sekundärstruktur-zusammensetzung ist, einschließlich Amyloid-beta-Peptiden und Tau-Protein (assoziiert mit der Alzheimer-Krankheit), Alpha-Synuclein (assoziiert mit der Parkinson-Krankheit), Prion-Protein (assoziiert mit der Creutzfeldt-Jakob-Krankheit) oder Huntingtin-Protein (assoziiert mit Chorea Huntington); und/oder
(ii) die blockierende Substanz, die nicht mit dem Biomarker-Kandidatenprotein kreuzreagiert, ausgewählt ist aus Casein, Ethanolamin, L-Lysin, Polyethylenglykolen, Albuminen und Derivaten davon; und/oder
(iii) das Biomarker-Kandidatenprotein ein Amyloid-beta-Peptid ist und der Antikörper ein Antikörper ist, der spezifisch an das zentrale Epitop des Amyloid-beta-Peptids bindet, einschließlich Antikörper A8978; oder
(iv) das Biomarkerprotein Alpha-Synuclein ist und der Antikörper ein Antikörper ist, der spezifisch an das Alpha-Synuclein-Peptid ohne Konformationsspezifität bindet, einschließlich Antikörper 4B12 oder S5566.

6. Das Detektionssystem nach einem der Ansprüche 1 bis 5, wobei das System ferner einen IR-Detektor umfasst.

7. Verwendung des Infrarot-Detektionssystems nach einem der Ansprüche 1 bis 6 zur Bestimmung der Sekundärstruktur und gegebenenfalls der Quantität eines Biomarker-Kandidatenproteins, das Konformationsübergänge durchläuft, die mit dem Fortschreiten der Krankheit in einer komplexen Flüssigkeit einschließlich Körperflüssigkeiten verbunden sind, durch Differenzspektroskopie.

8. Verfahren zur Bestimmung der Sekundärstruktur und gegebenenfalls der Quantität eines Biomarker-Kandidatenproteins, das Konformationsübergänge durchläuft, die mit dem Fortschreiten einer Krankheit in einer komplexen Flüssigkeit verbunden sind, durch Differenzspektroskopie, umfassend die Schritte:
(a) Leiten eines Flusses potenzieller Kandidaten-Biomarkerproteine für den Rezeptor auf der Oberfläche des Infrarotsensors in der IR-Zelle, die das Infrarotsensorelement nach einem der Ansprüche 1 bis 6 umfasst;
(b) Aussenden eines IR-Strahls durch die Zelle mit dem abstimmbaren Quantenkaskadenlaser und Erhalten eines Infrarotspektrums davon; und
(c) Analysieren des erhaltenen Infrarotspektrums, um die Sekundärstruktur und gegebenenfalls die Quantität des Kandidaten-Biomarkerproteins zu bestimmen, indem das erhaltene Infrarotspektrum mit einem Spektrum des Kandidaten-Biomarkerproteins mit bekannter Sekundärstruktur und gegebenenfalls mit einer bekannten Konzentration davon verglichen wird.

9. Verfahren nach Anspruch 8, wobei
(i) das Verfahren ferner den Schritt (d) umfasst: Analysieren des erhaltenen Infrarotspektrums, um die Probe mit statistischen Methoden auf der Grundlage der Sekundärstruktur-zusammensetzung des Biomarkerkandidatenproteins zu klassifizieren;
und/oder
(ii) das Verfahren ferner den Schritt (e) umfasst: Regenerieren der Oberfläche des Infrarotelements durch Aufbringen einer Lösung eines freien Liganden für den Rezeptor;
und/oder
(iii) das in Schritt (b) erhaltene Spektrum ein ausreichendes Signal-Rausch-Verhältnis aufweist, um die Amid-I-Bande aufzulösen; und/oder
(iv) Schritt (c) umfasst das Analysieren der Verschiebung des Maximums der Amid-I-Bande des Biomarkerproteins, um die Sekundärstruktur des Biomarkerkandidatenproteins zu bestimmen; und/oder
(v) das Verfahren umfasst ferner parallel zur Infrarotanalyse den Nachweis durch ein anderes optisches Verfahren, einschließlich UV/Vis-Fluoreszenz, bei unterschiedlichen Wellenlängen; und/oder
(vi) das Verfahren kombiniert die Immuno-ATR-IR-Schwingungsspektroskopie mit paralleler Fluoreszenzspektroskopie; und/oder
(vii) das Verfahren eignet sich zur direkten Bestimmung von Biomarkerproteinen in Körperflüssigkeiten, einschließlich Liquor, Blut oder Serum, ohne Vorbehandlung;
und/oder
(viii) das Verfahren eignet sich zur separaten (in vitro) oder Online-Bestimmung des Biomarker-Protein-Kandidaten.

10. Verfahren nach Anspruch 8 oder 9, das zur Bestimmung des Fortschreitens einer Krankheit dient, bei dem ein Konformationsübergang eines Biomarker-Kandidatenproteins mit dem Fortschreiten der Krankheit assoziiert ist, wobei eine Verschiebung des Amid-I-Bandenmaximums des Biomarker-Proteins ein Klassifikator ist, der auf das Fortschreiten der Krankheit hinweist, vorzugsweise ein Schwellenwert-Klassifikator mit einem Wert von 1638-1648 cm⁻¹ ein Klassifikator ist, der auf das Fortschreiten der Krankheit hinweist.

11. Verfahren nach einem der Ansprüche 8 bis 10, das
(i) zur Bestimmung des Fortschreitens der Alzheimer-Krankheit mit Amyloid-beta als Kandidaten-Biomarkerprotein dient, wobei eine Abwärtsverschiebung des Amid-I-Bandenmaximums des Amyloid-beta-Peptids, vorzugsweise mit einem Schwellenwert innerhalb von 1638-1648 cm⁻¹, auf ein Fortschreiten der Alzheimer-Krankheit hinweist;
oder
(ii) zur Bestimmung des Fortschreitens der Parkinson-Krankheit mit Alpha-Synuclein als Kandidaten-Biomarkerprotein dient, wobei eine Abwärtsverschiebung des Amid-I-Bandenmaximums des Alpha-Synuclein-Peptids, vorzugsweise mit einem Schwellenwert innerhalb von 1638-1648 cm⁻¹, für das Fortschreiten der Parkinson-Krankheit indikativ ist.

## Revendications

1. Système de détection infrarouge pour l'analyse spectroscopique différentielle directe de la quantité et de la structure secondaire d'une protéine biomarqueur candidate subissant des transitions conformationnelles associées à une progression de maladie, ledit système de détection comprenant :
(a) un laser à cascade quantique réglable en tant que la source IR, et
(b) une cellule IR avec un élément capteur infrarouge qui comprend un élément de réflexion interne en germanium qui est de forme trapézoïdale ou parallélogrammatique, qui autorise plus d'un passage de la lumière infrarouge à travers l'élément de réflexion, et qui est transparent dans l'infrarouge avec un rapport signal-bruit suffisant pour détecter la bande d'amide I, et au moins un récepteur pour la protéine biomarqueur qui est un anticorps capable de se lier de manière spécifique et conformationnellement indépendante à la protéine biomarqueur candidate et qui est directement greffé à au moins une surface dudit élément de réflexion interne en germanium par silanisation avec des lieurs de silane courts ou par thiolation avec des lieurs de thiol courts, faisant réagir librement des groupes d'amine accessibles dudit au moins un récepteur avec des groupes amine-réactifs sur les lieurs de silane/thiol courts, et bloquant des groupes amine-réactifs restants sur les lieurs de silane/thiol courts avec une substance bloquante qui n'effectue pas de réaction croisée avec la protéine biomarqueur candidate.

2. Le système de détection infrarouge de la revendication 1, sachant que
(i) l'élément capteur convient en outre pour l'analyse parallèle par un autre procédé optique incluant une détection de fluorescence à différentes longueurs d'onde ;
et/ou
(ii) l'élément de réflexion interne est un monocristal de germanium, de préférence est un monocristal de germanium à découpe trapézoïdale.

3. Le système de détection infrarouge de la revendication 1 ou 2, sachant que les lieurs de silane et de thiol incluent des lieurs de silane et de thiol homogènes, des mélanges de lieurs de silane et des mélanges de lieurs de thiol, et ont une longueur de chaîne qui n'est pas supérieure à 20 atomes ou qui n'est pas supérieure à 15 atomes, de préférence
les lieurs de silane ont une des formules suivantes :
X₃Si-(CH₂)ₙ-Y-(CH₂)_{n'}-Z,
X₂R¹Si-(CH₂)ₙ-Y-(CH₂)_{n'}-Z
ou
X(R¹)₂Si-(CH₂)ₙ-Y-(CH₂)_{n'}-Z,
et les lieurs de thiol ont la formule suivante :
HS-(CH₂)ₙ-Y-(CH₂)_{n'}-Z,
sachant que X à chaque occurrence est sélectionné indépendamment parmi de l'halogène et un alkoxy en C₁₋₆, n est un entier de 1 à 10, n'est un entier de 1 à 5 ; R¹ à chaque occurrence est sélectionné indépendamment parmi un alkyle en C₁₋₆, Y est sélectionné parmi une liaison chimique, -O-, -CO-, -SO₂-, -NR²-, -S-, -SS-, -NR²CO-, - CONR²-, -NR²SO₂- et -SO₂NR²- (sachant que R² est H ou un alkyle en C₁₋₆), et Z est un groupe amine-réactif incluant -CO₂H, -SO₃H et des dérivés d'ester de ceux-ci.

4. Le système de détection infrarouge de la revendication 3, sachant que
(i) X dans les lieurs de silane est sélectionné indépendamment parmi des groupes alkoxy en C₁₋₆, de préférence parmi des groupes méthoxy et éthoxy, Y est - NHCO-, Z est -CO₂H ou un dérivé d'ester de celui-ci, et n est un entier de 1 à 5 et n'est un entier de 1 à 3, de préférence n est 3 et n'est 2 ; ou
(ii) Y dans les lieurs de thiol est une liaison chimique, Z est -CO₂H ou un dérivé d'ester de celui-ci, et n est un entier de 1 à 8 et n'est un entier de 1 à 5, de préférence n est 8 et n'est 4.

5. Le système de détection infrarouge de l'une quelconque des revendications 1 à 4, sachant que
(i) la protéine biomarqueur candidate subissant des transitions conformationnelles associées à une progression de maladie est un peptide amyloïdogénique ou un (poly-)peptide de composition de structure secondaire caractéristique dépendante de l'état de santé, incluant des peptides bêta-amyloïdes et une protéine tau (associée à la maladie d'Alzheimer), une alpha-synucléine (associée à la maladie de Parkinson), une protéine prion (associée à la maladie de Creutzfeldt-Jakob), ou une protéine huntingtine (associée à la chorée Huntington) ; et/ou
(ii) la substance bloquante n'effectuant pas de réaction croisée avec la protéine biomarqueur candidate est sélectionnée parmi la caséine, l'éthanolamine, la L-lysine, les glycols de polyéthylène, les albumines et les dérivés de ceux-ci ; et/ou
(iii) la protéine biomarqueur candidate est un peptide bêta-amyloïde et l'anticorps est un anticorps se liant spécifiquement à l'épitope central du peptide bêta-amyloïde, incluant l'anticorps A8978 ; ou
(iv) la protéine biomarqueur candidate est une alpha-synucléine et l'anticorps est un anticorps se liant spécifiquement au peptide alpha-synucléine sans spécificité conformationnelle, incluant l'anticorps 4B12 ou S5566.

6. Le système de détection de l'une quelconque des revendications 1 à 5, ledit système comprenant en outre un détecteur IR.

7. Utilisation du système de détection infrarouge de l'une quelconque des revendications 1 à 6 pour déterminer la structure secondaire, et facultativement la quantité, d'une protéine biomarqueur candidate subissant des transitions conformationnelles associées à une progression de maladie dans un fluide complexe incluant des fluides corporels par spectroscopie différentielle.

8. Procédé destiné à déterminer la structure secondaire, et facultativement la quantité, d'une protéine biomarqueur candidate subissant des transitions conformationnelles associées à une progression de maladie dans un fluide complexe par spectroscopie différentielle, comprenant les étapes :
(a) conduction, dans la cellule IR comprenant l'élément capteur infrarouge des revendications 1 à 6, d'un flux de protéines biomarqueurs candidates potentielles pour le récepteur sur la surface dudit capteur infrarouge ;
(b) soumission d'un faisceau IR à travers ladite cellule avec le laser à cascade quantique réglable et obtention d'un spectre infrarouge à partir de celui-ci ; et
(c) analyse du spectre infrarouge obtenu pour déterminer la structure secondaire, et facultativement la quantité, de la protéine biomarqueur candidate en comparant le spectre infrarouge obtenu avec un spectre de la protéine biomarqueur candidate avec structure secondaire connue, et facultativement avec une concentration connue de celle-ci.

9. Le procédé de la revendication 8, sachant que
(i) le procédé comprend en outre l'étape (d) : analyse du spectre infrarouge obtenu pour classifier l'échantillon avec des méthodes statistiques sur la base de la composition de structure secondaire de la protéine biomarqueur candidate ; et/ou
(ii) le procédé comprend en outre l'étape (e) : régénération de la surface de l'élément infrarouge par application d'une solution de ligand libre pour le récepteur ; et/ou
(iii) le spectre obtenu à l'étape (b) a un rapport signal-bruit suffisant pour résoudre la bande d'amide I ; et/ou
(iv) l'étape (c) comprend l'analyse du décalage du maximum de bande d'amide I de la protéine biomarqueur pour déterminer la structure secondaire de la protéine biomarqueur candidate ; et/ou
(v) le procédé comprend en outre, parallèlement à l'analyse infrarouge, une détection par un autre procédé optique, incluant la fluorescence UV/Vis, à différentes longueurs d'onde ; et/ou
(vi) le procédé combine la spectroscopie vibrationnelle immuno-ATR-IR avec la spectroscopie de fluorescence ; et/ou
(vii) le procédé convient pour déterminer directement des protéines biomarqueurs candidates dans des fluides corporels, incluant du fluide cérébrospinal, du sang ou du sérum, sans prétraitement ; et/ou
(viii) le procédé convient pour la détermination séparée (*in-vitro*) ou en ligne de la protéine biomarqueur candidate.

10. Le procédé de la revendication 8 ou 9, qui est destiné à déterminer une progression d'une maladie, dans lequel une transition conformationnelle d'une protéine biomarqueur candidate est associée à une progression de maladie, sachant qu'un décalage du maximum de bande d'amide I de la protéine biomarqueur est un classificateur indicatif de la progression de la maladie, de préférence un classificateur seuil avec une valeur de 1638-1648 cm⁻¹ est un classificateur indicatif de la progression de la maladie.

11. Le procédé de l'une quelconque des revendications 8 à 10, qui est destiné à
(i) déterminer une progression de la maladie d'Alzheimer avec du bêta-amyloïde comme protéine biomarqueur candidate, sachant qu'un décalage vers le bas du maximum de bande d'amide I du peptide bêta-amyloïde, de préférence avec une valeur seuil comprise dans 1638-1648 cm⁻¹, est indicatif d'une progression de la maladie d'Alzheimer ; ou
(ii) déterminer une progression de la maladie de Parkinson avec de l'alpha-synucléine comme protéine biomarqueur candidate, sachant qu'un décalage vers le bas du maximum de bande d'amide 1 de l'alpha-synucléine, de préférence avec une valeur seuil comprise dans 1638-1648 cm⁻¹, est indicatif de la progression de la maladie de Parkinson.
